(19) Europäisches Patentamt / European Patent Office / Office européen des brevets

(11) **EP 4 017 355 B1**

(12) **EUROPEAN PATENT SPECIFICATION**

(45) Date of publication and mention of the grant of the patent:
**06.11.2024 Bulletin 2024/45**

(21) Application number: **20764468.3**

(22) Date of filing: **21.08.2020**

(51) International Patent Classification (IPC):
*A61B 5/11* (2006.01)     *A61B 5/00* (2006.01)
*A61M 1/00* (2006.01)     *G06T 7/246* (2017.01)
*A61F 13/00* (2024.01)

(52) Cooperative Patent Classification (CPC):
**A61B 5/1127; A61B 5/1121; A61B 5/4836;**
**A61B 5/6898; A61M 1/85; A61M 1/915;**
**G06T 7/251;** A61F 13/00051; A61F 13/05;
A61M 1/912; A61M 1/92; G06T 2207/30004

(86) International application number:
**PCT/IB2020/057868**

(87) International publication number:
**WO 2021/038408 (04.03.2021 Gazette 2021/09)**

(54) **SYSTEMS FOR DETERMINING AND TRACKING RANGE OF MOTION OF A JOINTED LIMB**

SYSTEME ZUR BESTIMMUNG UND VERFOLGUNG DES BEWEGUNGSBEREICHS EINES GELENKSCHENKELS

SYSTÈMES DE DÉTERMINATION ET DE SUIVI DE LA PLAGE DE MOUVEMENT D'UN MEMBRE ARTICULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.08.2019 US 201962890804 P**

(43) Date of publication of application:
**29.06.2022 Bulletin 2022/26**

(73) Proprietor: **Solventum Intellectual Properties Company**
**Maplewood, MN 55144 (US)**

(72) Inventors:
 • **REHBEIN, Jonathan G.**
 **San Antonio, Texas 78265 (US)**
 • **RANDOLPH, Larry Tab**
 **San Antonio, Texas 78265 (US)**
 • **MERCER, David R.**
 **San Antonio, Texas 78265 (US)**

 • **KAZALA, Richard**
 **San Antonio, Texas 78265 (US)**
 • **PERKINS, Luke**
 **San Antonio, Texas 78265 (US)**

(74) Representative: **Simmons & Simmons**
**City Point**
**One Ropemaker Street**
**London EC2Y 9SS (GB)**

(56) References cited:
 **WO-A1-2019/096828     US-A1- 2016 220 175**
 **US-A1- 2017 258 526     US-B1- 10 121 066**

 • **SU F C ET AL: "Movement of finger joints induced by synergistic wrist motion", CLINICAL BIOMECHANICS, BUTTERWORTH SCIENTIFIC LTD, GUILDFORD, GB, vol. 20, no. 5, 1 June 2005 (2005-06-01), pages 491 - 497, XP027795131, ISSN: 0268-0033, [retrieved on 20050601]**

**Description**

BACKGROUND

The present disclosure relates generally to a wound therapy system, and more particularly to measuring range of motion during healing progression of a wound.

Negative pressure wound therapy (NPWT) is a type of wound therapy that involves applying a negative pressure to a wound site to promote wound healing. Some wound treatment systems apply negative pressure to a wound using a pneumatic pump to generate the negative pressure and flow required. Recent advancements in wound healing with NPWT involve applying topical fluids to wounds to work in combination with NPWT. However, it can be difficult to measure range of motion accurately and precisely as the wound heals.

US2016/220175A1 discusses a system for providing information to a patient and receiving information from the patient before and/or after a medical or surgical procedure. The system may include a joint motion sensor system, which may include a first sensor device for coupling with the patient above a joint and including a first transmitter for transmitting sensed data from the first sensor device, and a second sensor device for coupling with the patient below the joint and including a second transmitter for transmitting sensed data from the second sensor device.

US2017/258526A1 discusses devices and methods for performing a surgical step or surgical procedure with visual guidance using an optical head mounted display.

US10121066B1 discusses a method of calculating the stress on a joint by determining when a joint angle is outside the normal range of motion for the joint. Applications include analysis of elbow joint stress for baseball pitchers, for example to mitigate the risk of injury to the ulnar collateral ligament (UCL). During a movement such as a baseball pitch, sensor data is collected to track the position, orientation, or motion of body segments; joint angles are derived from this sensor data. Joint angles are then compared to a range of motion, which may be measured for each individual. Joint stress contributions from movements that exceed the range of motion may be combined over time to calculate cumulative joint fatigue.

Su et al., "Movement of finger joints induced by synergistic wrist motion", Clinical Biomechanics, Volume 20, Issue 5, June 2005 discusses an experiment in which the relative joint positions of the hand and wrist of subjects were measured using a three-dimensional motion analysis system with external retroreflective markers 2 mm in diameter placed on the dorsal surface of the hand.

SUMMARY

There is provided a system for calculating range of motion of a patient's jointed limb according to claim 1. Optional features are set out in the dependent claims.

BRIEF DESCRIPTION OF THE DRAWINGS

FIG. 1 is a perspective view of a patient's joint with a NPWT system and three locators positioned about the patient's joint, according to some embodiments.

FIG. 2 is a diagram of a patient's mobile device displaying an image of the patient's joint with centerlines extending through the locators, and an angle notification, according to some embodiments.

FIG. 3 is a perspective view of a patient's joint with a NPWT system and four locators positioned about the patient's joint, according to some embodiments.

FIG. 4 is a perspective view of the patient's joint of FIG. 3, according to some embodiments.

FIG. 5 is a diagram of a patient's mobile device displaying an image of the patient's joint with centerlines extending through the locators, according to some embodiments.

FIG. 6 is a block diagram of a patient's mobile device configured to capture image data of the patient's joint, determine positions of the locators, calculate range of motion of the patient's joint, generate and display reports, and communicate with a clinician device, according to some embodiments.

FIGS. 7-8 are drawings that illustrate skew of the image of the locators due to orientation between the patient's mobile device and the locators for circular locators, according to some embodiments.

FIGS. 9-10 are drawings that illustrate skew of the image of the locators due to orientation between the patient's mobile device and the locators for square locators, according to some embodiments.

FIG. 11 is a drawing of three locators and centerlines that extend through the locators of an initially captured or baseline image, according to some embodiments.

FIG. 12 is a drawing of the three locators and centerlines of FIG. 11 of a later captured image, according to some embodiments.

FIG. 13 is a diagram of a spherical coordinate system between the patient's mobile device and the patient's joint that illustrates azimuth and elevation angles, according to some embodiments.

FIG. 14 is a flow diagram of a process for capturing image data and determining a range of motion of a patient's joint based on the captured image data, according to some embodiments.

FIG. 15 is a flow diagram of a process for configuring a patient's mobile device to analyze image data to determine range of motion values and to generate range of motion progress reports and communicate with a clinician device, according to some embodiments.

FIG. 16 is a flow diagram of a process for offsetting or adjusting the range of motion of the patient's joint of FIG. 14 to account for relative orientation between the patient's mobile device and the patient's joint, according to some embodiments.

FIG. 17 is a graph of range of motion of a patient's joint over time, according to some embodiments.

FIG. 18 is a drawing of a mobile device displaying a range of motion progress report, according to some embodiments.

FIG. 19 is a table of flexed, extended, and range of motion angular values, as well as recordation dates, percent improvement, and total percent improvement that can generated as a range of motion report, according to some embodiments.

FIG. 20 is a flow diagram of a process for generating and displaying a range of motion progress report, according to some embodiments.

FIG. 21 is a flow diagram of a process for notifying a patient to capture image data of the patient's joint, according to some embodiments.

FIG. 22 is a front view of a wound dressing according to some embodiments.

FIG. 23 is a perspective view of the wound dressing of FIG. 22 according to an exemplary embodiment.

FIG. 24 is an exploded view of the wound dressing of FIG. 22 according to an exemplary embodiment.

FIG. 25 is a side cross-sectional view of the wound dressing of FIG. 22 adhered to a patient according to an exemplary embodiment.

FIG. 26 is a perspective view of a manifold layer of the wound dressing of FIG. 22 according to an exemplary embodiment.

FIG. 27 is a block diagram of the NPWT system of FIG. 1 including a therapy device coupled to a wound dressing via tubing, according to some embodiments.

FIG. 28 is a block diagram illustrating the therapy device of FIG. 27 in greater detail when the therapy device operates to draw a vacuum within a negative pressure circuit, according to an exemplary embodiment.

FIG. 29 is a block diagram illustrating the therapy device of FIG. 27 in greater detail when the therapy device operates to vent the negative pressure circuit, according to an exemplary embodiment.

FIG. 30 is a block diagram illustrating the therapy device of FIG. 27 in greater detail when the therapy device uses an orifice to vent the negative pressure circuit, according to an exemplary embodiment.

FIG. 31 is a block diagram illustrating the therapy device of FIG. 27 in greater detail when the therapy device operates to deliver instillation fluid to the wound dressing and/or a wound, according to an exemplary embodiment.

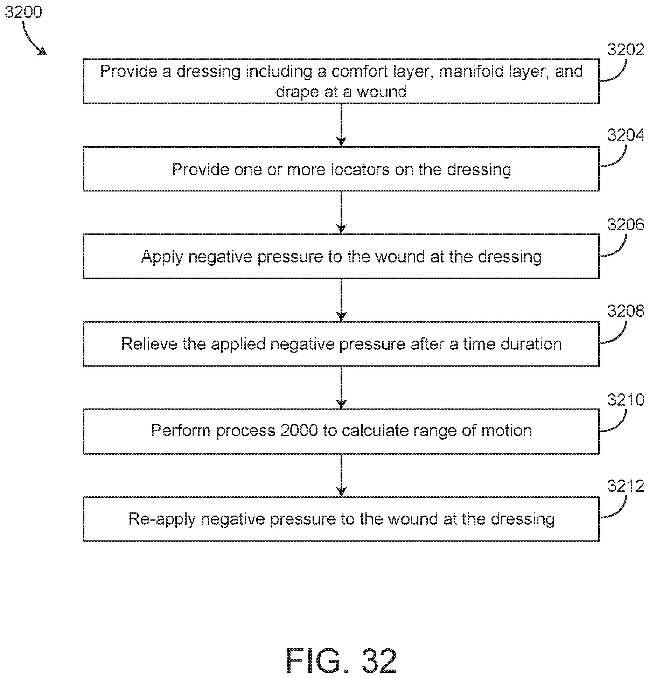

FIG. 32 is a flow diagram of a process for performing negative pressure wound therapy and calculating a range of motion of a jointed limb, according to some embodiments.

## DETAILED DESCRIPTION

### Overview

Referring generally to the FIGURES, systems and methods for measuring range of motion of a patient's joint are shown. A smartphone or a personal computer device can be used with an installed mobile application for measuring the range of motion of the patient's joint. Three or four locators (e.g., dots, squares, reflective material, etc.) can be pre-affixed to a dressing, a drape, or the patient's skin. The patient can be reminded at regular time intervals to measure the range of motion. When the patient measures the range of motion, images of the joint in both the fully flexed and the fully extended configuration are recorded. The mobile application identifies positions of the locators, generates lines that extend through the locators and determines angles between the lines. The mobile application determines angles for both the fully flexed image and the fully extended image. The mobile application then determines a difference between the fully extended angle and the fully flexed angle as the range of motion. The mobile application can configure the smartphone to communicate with a clinician device. The mobile application may generate reports and operate a screen of the smartphone to display the reports. The mobile application can also store range of motion measurements throughout healing of the patient's wound. The mobile application can provide reports (e.g.,, graphs, tabular data, analysis, etc.) to the clinician device.

The mobile application can also perform a calibration technique to identify position and orientation of the smartphone relative to the patient's limb. The mobile application can analyze the images to determine orientation of the smartphone relative to the patient's limb. The mobile application offsets the range of motion to account for orientation of the smartphone relative to the patient's limb. Advantageously, the systems and methods described herein can enable a patient to measure the range of motion of their limb at home. The range of motion can be provided to a remotely positioned clinician device for clinician monitoring, analysis, and checkups.

**Tracking System**

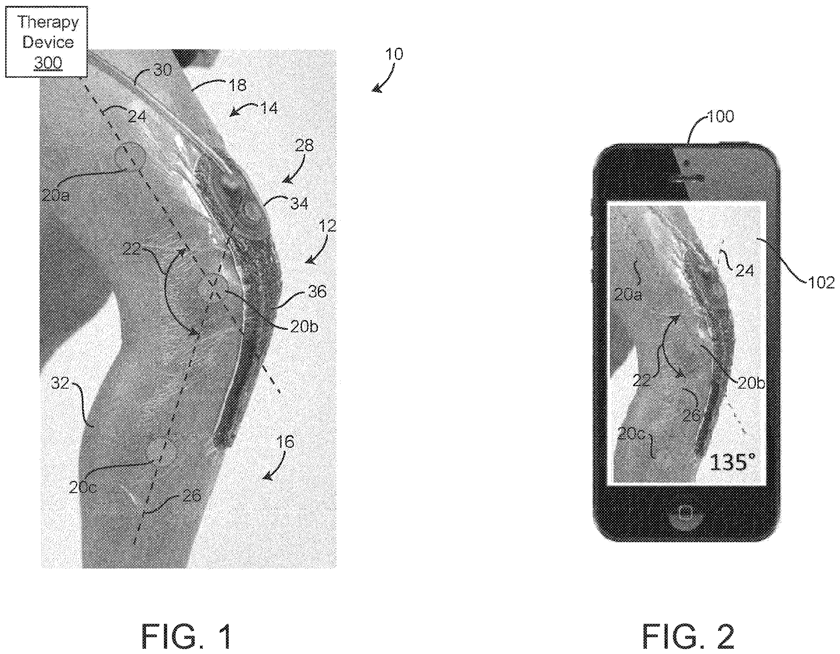

Referring now to FIG. 1, a system 10 for tracking range of motion of a patient's limb is shown. System 10 is shown applied at a patient's knee joint. However, system 10 can be applied at any patient joint (e.g., an elbow, a wrist, etc.) that includes a first limb and a second limb that are jointedly connected.

System 10 includes a negative pressure wound therapy (NPWT) system 28 applied to a patient's wound, according to some embodiments. NPWT system 28 can include a dressing 36 that substantially covers and seals the patient's wound. Dressing 36 can be adhered and sealed to patient's skin 32 and covers the patient's wound. Dressing 36 can be a foam dressing that adheres to the patient's skin 32. NPWT system 28 can include a therapy device 300 (e.g., a NPWT device) that fluidly couples with an inner volume of the patient's wound. Therapy device 300 can be configured to draw a negative pressure at the patient's wound. Therapy device 300 can be fluidly coupled with the patient's wound through conduit, tubing, medical tubing, flexible tubing, etc., shown as tubular member 30. Tubular member 30 can include an inner volume for drawing a negative pressure at the patient's wound. Tubular member 30 can include an inner volume for providing instillation fluid (e.g., a saline solution) to the patient's wound.

Tubular member 30 can be fluidly coupled with therapy device 300 at a first end (not shown) and with the patient's wound at a second end. In some embodiments, tubular member 30 fluidly couples with the patient's wound (e.g., an inner volume of the patients wound) through a connector 34. Connector 34 can be sealed on an exterior surface of dressing 36 and can be fluidly coupled with inner volume between the patient's skin/wound and dressing 36. Tubular member 30 is configured to facilitate drawing negative pressure at the wound site.

In use, a drape 18 is adhered to patient's skin 32 and covers substantially the entire dressing 36. Drape 18 can be a thin film, a plastic film, a plastic layer, etc., that adheres to an exterior surface of dressing 36 and skin surrounding dressing 36 (e.g., periwound skin).

Drape 18 can seal with the patient's skin 32 to facilitate a sealed fluid connection between tubular member 30 (e.g., and the NPWT device) and the patient's wound or surgical incision.

Trackers, locators, dots, etc., shown as locators 20 are applied to drape 18. Locators 20 can be printed on drape 18, adhered to drape 18 after drape 18 is applied, or adhered to the patient's skin 32 before drape 18 is applied. For example, if drape 18 is transparent or translucent, locators 20 can be applied to the patient's skin 32 before drape 18 is applied. Drape 18 can then be applied over locators 20 which are still visible through drape 18. In other embodiments, locators 20 are applied onto an exterior surface of drape 18 after drape 18 is applied to skin 32. In still other embodiments, locators 20 are applied to the patient's skin 32 surrounding drape 18. For example, locators 20 can be applied to the patient's skin 32 at various locations along a perimeter of drape 18.

Locators 20 can be any visual indicator that can be tracked, located, etc., to determine range of motion of the patient's limb. Three locators 20 are applied to the patient's limb. One locator 20b is applied to joint 12 of the patient's limb, while another locator 20a is applied at upper limb 14, and another locator 20c is applied at lower limb 16. Locators 20 can be applied to any joint or hingedly coupled limbs of a patient. For example, FIG. 1 shows locator 20a applied to the patient's thigh (e.g., upper limb 14), locator 20b applied to the patient's knee (e.g., joint 12), and locator 20c applied to the patient's calf (e.g., lower limb 16). In other embodiments, locator 20a is applied to a patient's upper arm, locator 20b is applied to the patient's elbow, and locator 20c is applied to the patient's forearm. Locators 20 are visual indicators that can be identified through image analysis to determine approximate location of each of locators 20 and determine angle 22.

Angle 22 is formed between centerline 24 and centerline 26. Centerline 24 extends between a center of locator 20a and a center of locator 20b (the locator that is positioned at joint 12). Centerline 26 extends between a center of locator 20c and a center of locator 20b (the locator that is positioned/applied at joint 12). Centerlines 24 and 26 define angle 22 that indicates a degree of extension or flexion of the jointed limbs of the patient. Angle 22 is calculated/determined by identifying locations/positions of locators 20, adding centerlines 24 and 26 through locators 20, and calculating angle 22 therebetween centerlines 24 and 26.

Referring now to FIG. 2, a personal computer device (e.g., a smartphone, a tablet, a laptop computer, etc.), shown as smartphone 100 displays an image of the patient's jointed limb and angle 22. Smartphone 100 also displays a calculated value of angle 22 (e.g., 135 degrees as shown in the lower right corner of touchscreen 102). Smartphone 100 includes a display screen, a user interface, a touchscreen, etc., shown as touchscreen 102. Touchscreen 102 can be configured to display imagery, information, augmented reality images, etc., to a patient or a user. In some embodiments, touchscreen 102 is configured to receive user inputs (e.g., commands to take a picture, commands to calculate angle 22, etc.).

Smartphone 100 can perform an angle or range of motion analysis to determine angle 22. In some embodiments, smartphone 100 can download and install an application (e.g., a mobile app) that configures smartphone 100 to calculate angle 22. The mobile app can use various sensory inputs of smartphone 100 to obtain image data and calculate angle 22. For example, smartphone 100 can include a camera, an accelerometer, touchscreen 102, a user interface, buttons, wireless communications, etc. The application can use any of the sensory inputs from the user interface, accelerometer, camera, touchscreen 102, buttons, wireless communications, etc., to determine/identify the locations of locators 20 and to calculate a value of angle 22. The application may configure smartphone 100 to perform any of the functionality, techniques, processes, etc., locally (e.g., via a processor and/or processing circuit that is locally disposed within smartphone 100). The application can configure smartphone 100 to provide image data and/or any other sensor data to a remote server, and the remote server performs any of the functionality, techniques, processes, etc., described herein to determine locations of locators 20 and to calculate a value of angle 22. In some embodiments, angle 22 is referred to as angle $\theta$.

The application can prompt the patient to capture imagery data (e.g., take a picture) at a fully flexed state and a fully extended state. For example, the application can prompt the patient to fully flex their jointed limb and record image data. The application can then prompt the patient to fully extend their jointed limb and record image data. In some embodiments, the application prompts the patient to record fully flexed and fully extended image data via touchscreen 102. For example, the application can provide notifications, alerts, reminders, etc., that the patient should capture both fully flexed and fully extended image data. Smartphone 100 and/or a remote server can be configured to perform a process, algorithm, image analysis technique, etc., to determine a value of angle 22 in both the fully flexed position and the fully extended position. The fully extended value of angle 22 can be referred to as $\theta_{extend}$ and the fully flexed value of angle 22 is referred to as $\theta_{flexed}$. $\theta_{extend}$ can be determined by the application (e.g., locally by a processor and/or processing circuit of smartphone 100, remotely by a remote device, server, collection of devices, etc.) based on the fully extended image data. $\theta_{flexed}$ can be determined by the application similar to $\theta_{extend}$ based on the fully flexed image data.

The value of angle 22 can be determined by performing an image analysis technique to determine locations of locators 20. For example, the application can configure smartphone 100 to identify locations of locators 20. In some embodiments, if three locators (e.g., 20a, 20b, and 20c) are used, the application identifies loca-

tions $p_1$, $p_2$, and $p_3$ of locators 20. The identified locations $p$ can include an x-position coordinate, and a y-position coordinate. For example, the application can determine that locator 20a has a location $p_1 = \{x_1\ y_1\}$, that locator 20b has a location $p_2 = \{x_2\ y_2\}$, and that locator 20c has a location $p_3 = \{x_3\ y_3\}$. The application can use the determined locations to generate centerlines 24 and 26. For example, centerline 24 can be determined based on the identified location of locator 20a, and the identified location of locator 20b. The application can be configured to use the identified locations of locator 20a and locator 20b to determine a linear line

that extends through both locator 20a and locator 20b. For example, the application can determine centerline 24 in point-point form based on the identified locations of locators 20a and 20b as:

$$y - y_1 = \frac{y_2 - y_1}{x_2 - x_1}(x - x_1)$$

according to some embodiments.

The application can also be configured to determine centerline 24 and/or centerline 26 in point-slope form. In some embodiments, the application determines vectors (e.g., unit vectors) in Cartesian form, polar form, etc. The application can determine a value of the angle 22 based on the equations, vectors, etc., of centerline 24 and centerline 26. The application can determine an intersection location where centerline 24 and centerline 26 intersect, and determine angle 22 between centerline 24 and centerline 26 at the intersection location.

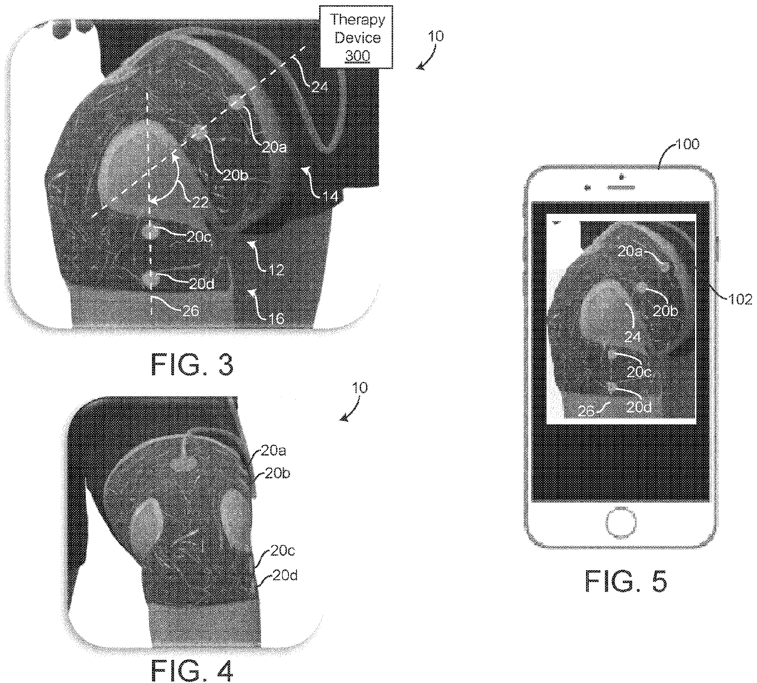

Referring now to FIGS. 3-5, system 10 can include four locators 20. In some embodiments, a first set of two locators 20 are positioned on the patient's upper limb 14 (above the joint 12), while a second set of two locators 20 are positioned on the patient's lower limb 16 (below the joint 12). Centerline 24 can be determined based on the identified locations of the first set of locators 20 (e.g., locator 20a and locator 20b). For example, centerline 24 can be determined by identifying the locations $p_1$ and $p_2$ of locator 20a and locator 20b, respectively, and generating a linear line (e.g., centerline 24) that extends through the identified location of locator 20a and locator 20b. Centerline 26 can be determined similarly to centerline 24 (e.g., by identifying the locations, $p_3$ and $p_4$ of locators 20c and 20d) and generating a linear line that extends through the identified locations of locators 20c and 20d. A value of angle 22 can be determined based on the generated centerlines 24 and 26. For example, the application can generate equations of centerline 24 and centerline 26 and determine the angle 22 formed by the intersection of centerlines 24 and 26.

In some embodiments, using four locators 20a-d provides a more accurate measurement of angle 22. For example, the precision, repeatability, accuracy, reliability, etc., of the value of angle 22 can be improved by using four locators 20a-d. Four locators 20 or three locators 20 can be used as preferred by a clinician.

## Controller

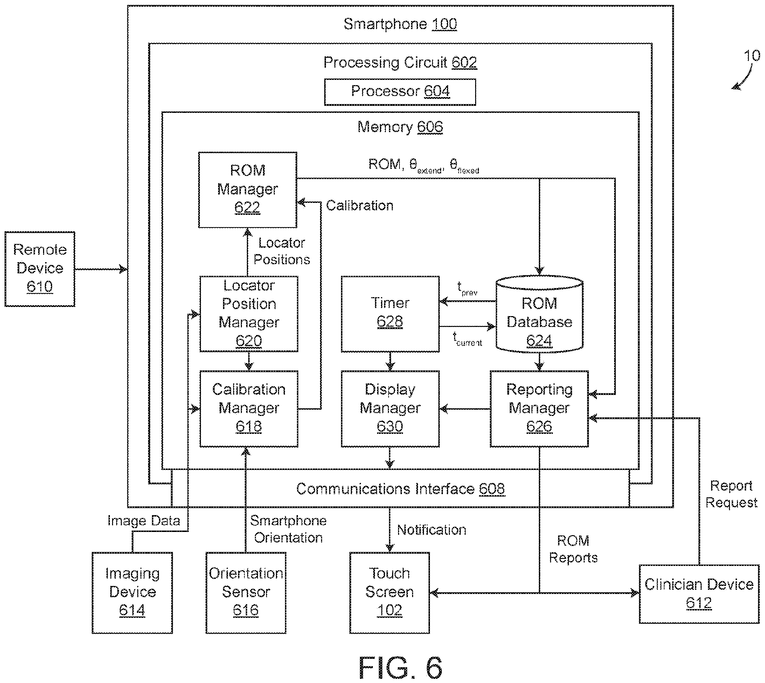

Referring now to FIG. 6, system 10 is shown in greater detail. Smartphone 100 is configured to receive image data from an imaging device, a camera, a digital camera, etc., shown as imaging device 614. In some embodiments, imaging device 614 is a component of smartphone 100. Smartphone 100 uses the image data received from imaging device 614 to determine positions of locators 20, and to determine the angles $\theta_{extend}$, $\theta_{flexed}$, and a range of motion of joint 12. Any of the functionality of smartphone 100 can performed by a remote device, a remote server, a remote network, etc. For example, smartphone 100 can wirelessly connect with a remote device and provide the image data to the remote device. The remote device can perform any of the functionality of smartphone 100 as described herein to determine range of motion of joint 12 based on the image data. In other embodiments, some of the functionality of smartphone 100 is performed by a remote device (e.g., determining the position of locators 20, performing calibration processes, etc.) and some of the functionality of smartphone 100 is performed locally (e.g., on a processing circuit of smartphone 100).

Referring still to FIG. 6, smartphone 100 is shown to include a communications interface 608. Communications interface 608 may facilitate communications between smartphone 100 and other applications, devices, components, etc. (e.g., imaging device 615, orientation sensor 616, touchscreen 102, clinician device 612, remote device 610, etc.) for allowing receiving and sending data. Communications interface 608 may also facilitate communications between smartphone 100 and remote device 610 or another smartphone.

Communications interface 608 can be or include wired or wireless communications interfaces (e.g., jacks, antennas, transmitters, receivers, transceivers, wire terminals, etc.) for conducting data communications with clinician device 612 or other external systems or devices. In various embodiments, communications via communications interface 608 can be direct (e.g., local wired or wireless communications) or via a communications network (e.g., a WAN, the Internet, a cellular network, Bluetooth, etc.). For example, communications interface 608 can include an Ethernet card and port for sending and receiving data via an Ethernet-based communications link or network. In another example, communications interface 608 can include a Wi-Fi transceiver for communicating via a wireless communications network. In another example, communications interface 608 can include cellular or mobile phone communications transceivers. In one embodiment, communications interface 608 is a power line communications interface. In other embodiments, communications interface 608 is an Ethernet interface.

Still referring to FIG. 6, smartphone 100 is shown to include a processing circuit 602 including a processor 604 and memory 606. Processing circuit 602 can be communicably connected to communications interface 608 such that processing circuit 602 and the various components thereof can send and receive data via communications interface 608. Processor 604 can be implemented as a general purpose processor, an application specific integrated circuit (ASIC), one or more field programmable gate arrays (FPGAs), a group of processing components, or other suitable electronic processing components.

Memory 606 (e.g., memory, memory unit, storage device, etc.) can include one or more devices (e.g., RAM, ROM, Flash memory, hard disk storage, etc.) for storing data and/or computer code for completing or facilitating the various processes, layers and modules described in the present application. Memory 606 can be or include volatile memory or non-volatile memory. Memory 606 can include database components, object code components, script components, or any other type of information structure for supporting the various activities and information structures described in the present application. According to some embodiments, memory 606 is communicably connected to processor 604 via processing circuit 602 and includes computer code for executing (e.g., by processing circuit 602 and/or processor 604) one or more processes described herein.

In some embodiments, the functionality of smartphone 100 is implemented within a single computer (e.g., one server, one housing, one computer, etc.). In various other embodiments the functionality of smartphone 100 can be distributed across multiple servers or computers (e.g., that can exist in distributed locations).

Memory 606 includes calibration manager 618, locator position manager 620, and range of motion (ROM) manager 622, according to some embodiments. Calibration manager 618, locator position manager 620, and ROM manager 622 can be configured to perform visual imaging processes to determine $\theta_{extend}$ and $\theta_{flexed}$. Calibration manager 618, locator position manager 620, and ROM manager 622 can be configured to receive image data from imaging device 614 to determine $\theta_{extend}$ and $\theta_{flexed}$.

In some embodiments, locator position manager 620 is configured to receive image data from imaging device 614. Locator position manager 620 can receive image data for both a fully flexed position of joint 12 and a fully extended position of joint 12. Locator position manager 620 can receive real time image data from imaging device 614. Locator position manager 620 can receive an image file (e.g., a jpeg file, a .png file, a .bmp file, etc.) from imaging device 614.

Locator position manager 620 is configured to perform an imaging processing technique to identify the positions of locators 20. Locator position manager 620 determines the positions of locators 20 based on the shape of the locators 20. The position manager 620 may determine the positions of locators 20 based on any of color of locators 20, brightness of locators 20, contrast of locators 20, etc. For example, locator position manager 620 can use Kernel-based tracking, Contour tracking, etc., or any other image analysis technique to determine the positions of locators 20. Locator position manager 620 can use a neural network technique (e.g., a convolutional neural network) to identify positions of locators 20 in the image file. Locator position manager 620 can be configured to use a Kalman filter, a particle filter, a Condensation algorithm, etc., to identify the positions of locators 20. Locator position manager 620 can also use an object detection technique to identify the position of locators 20. For example, locator position manager 620 can use a region-based convolutional neural network (RCNN) to identify the positions of locators 20.

Locator position manager 620 can determine the positions $p_i$ of any of locators 20 and provide the determined positions $p_i$ to ROM manager 622. In some embodiments, the determined positions of locators 20 are Cartesian coordinates (e.g., x and y positions of each of locators 20) relative to a coordinate system (e.g., relative to a corner of the image, relative to a center of the image, relative to a location of the image, etc.).

Locator position manager 620 can analyze both the fully flexed image and the fully extended image data concurrently or independently. For example, locator position manager 620 may first receive the fully flexed image data and determine the positions of locators 20, and provide the positions of locators 20 to ROM manager 622, then receive the fully extended image data and determine the positions of locators 20 and provide the positions of locators 20 to ROM manager 622. In some embodiments, locator position manager 620 receive both the fully flexed and the fully extended image data, and identifies the positions of locators 20 for both images concurrently.

Locator position manager 620 can generate a first set $P_{flex}$ of position data of locators 20, and a second set $P_{extend}$ of position data of locators 20. In some embodiments, the first set $P_{flex}$ includes the identified positions of locators 20 for the fully flexed image data and the second set $P_{extend}$ includes the identified positions of locators 20 for the fully extended image data. For example, $P_{flex}$ may have the form $P_{flex} = [p_1 \, p_2 \, ... \, p_n]$ where n is the number of locators 20, and $p_i$ is the position data of an ith locator 20. Likewise, $P_{extend}$ may have the form $P_{extend} = [p_1 \, p_2 \, ... \, p_n]$.

If three locators 20 are used (as shown in FIGS. 1-2), $P_{flex} = [p_1 \, p_2 \, p_3]$ and $P_{extend} = [p_1 \, p_2 \, p_3]$. If four locators 20 are used (as shown in FIGS. 3-5), $P_{flex} = [p_1 \, p_2 \, p_3 \, p_4]$ and $P_{extend} = [p_1 \, p_2 \, p_3 \, p_4]$.

Locator position manager 620 provides the positions of locators 20 (e.g., $P_{flex}$ and $P_{extend}$) to ROM manager 622. ROM manager 622 is configured to determine $\theta_{extend}$ and $\theta_{flexed}$ and a range of motion $\theta_{ROM}$ of joint 12 based on the positions of locators 20. In some embodiments, ROM manager 622 is configured to generate centerline 24 and centerline 26 based on the positions of locators 20. Centerline 24 and centerline 26 can be linear lines that extend between corresponding positions of locators 20.

If three locators 20 are used, ROM manager 622 can generate centerline 24 between locator 20a and locator 20b. For example, ROM manager 622 can use the positions $p_1$ and $p_2$ to generate centerline 24. In some embodiments, if the positions are Cartesian coordinates, ROM manager 622 generates centerline 24 using point-point form:

$$y = \frac{y_2 - y_1}{x_2 - x_1}(x - x_1) + y_1$$

where $x_1$ is the x-position of locator 20a (or locator 20b), $y_1$ is the y-position of locator 20a (or locator 20b), $x_2$ is the x-position of locator 20b (or locator 20a), and $y_2$ is the y-position of locator 20b (or locator 20a) as identified/determined by locator position manager 620.

ROM manager 622 can similarly generate centerline 26 through locators 20b and locators 20c using point-point form:

$$y = \frac{y_3 - y_2}{x_3 - x_2}(x - x_2) + y_2$$

where $y_3$ is the y-position of locator 20c, and $x_3$ is the x-position of locator 20c as determined by locator position manager 620.

In some embodiments, ROM manager 622 is configured to use the equations of centerline 24 and centerline 26 to determine a value of angle 22. ROM manager 622 can be configured to determine the value of angle 22 based on the positions of locators 20. ROM manager 622 can determine an angle between centerline 24 and a horizontal or vertical axis, and an angle between centerline 26 and a horizontal or vertical axis. In some embodiments, ROM manager 622 uses the equation:

$$\theta = \tan^{-1}\frac{|y_1 - y_2|}{|x_1 - x_2|} + \tan^{-1}\frac{|y_2 - x_3|}{|x_2 - x_3|}$$

to determine angle 22 ($\theta$), where $y_1$ is the y-position of locator 20a, $x_1$ is the x-position of locator 20a, $y_2$ is the y-position of locator 20b, $x_2$ is the x-position of locator 20b, $y_3$ is the y-position of locator 20c, and $x_3$ is the x-position of locator 20c.

In some embodiments, ROM manager 622 uses the equation:

$$\theta = \tan^{-1}\frac{|y_1 - y_3|}{|x_1 - x_3|}$$

to determine angle 22 ($\theta$).

In some embodiments, if four locators 20 are used, locator position manager 620 determines a position of a point of intersection (POI) of centerline 24 and 26. ROM manager 622 can determine centerline 24 and 26 using the techniques described above to generate linear equations. ROM manager 622 can generate a line that extends between locator 20a ($p_1$) and locator 20b ($p_2$) as centerline 24, and a line that extends between locator 20c ($p_3$) and locator 20d ($p_4$) as centerline 26. ROM manager 622 can set the equations of centerlines 24 and 26 equal to each other and solve for an x or y position of POI. In some embodiments, the determined value of the x or y position is input into the equation of centerline 24 or centerline 26 to determine the position of the POI.

ROM manager 622 then uses the equations of centerline 24 and 26 and the POI to determine angle 22. In some embodiments, ROM manager 622 uses trigonometric identities, the Pythagorean theorem, etc., to determine angle 22 based on the equations of centerline 24 and 26, the POI, and the positions of locators 20a-d.

ROM manager 622 can use any of the techniques, processes, methods, etc., described in greater detail hereinabove to determine the value of angle 22. ROM manager 622 can analyze both the fully flexed image data and the fully extended image data to determine values of angle 22. The value of angle 22 determined by ROM manager 622 based on the fully flexed image data is $\theta_{flexed}$, and the value of angle 22 determined by ROM manager 622 based on the fully extended image data is $\theta_{extend}$.

In some embodiments, ROM manager 622 uses $\theta_{flexed}$ and $\theta_{extend}$ to determine $\theta_{ROM}$. $\theta_{ROM}$ is an angular amount that the patient can flex or extend joint 12 from the fully extended to the fully flexed position. In some embodiments, ROM manager 622 is configured to determine $\theta_{ROM}$ using the equation: $\theta_{ROM} = \theta_{extend} - \theta_{flexed}$.

ROM manager 622 can provide the fully extended angle $\theta_{extend}$, the fully flexed angle $\theta_{flexed}$, and the range of motion angle $\theta_{ROM}$ to ROM database 624. ROM database 624 can be a local database (e.g., memory 606 of smartphone 100), or a remote database (e.g., a remote server) that is configured to wirelessly communicate with smartphone 100. In some embodiments, ROM database 624 is configured to store any of the received angular values. ROM database 624 can also configured to store a time, date, location, etc., at which the image data is captured, a time and date of when the angular values are calculated, etc. ROM database 624 can retrieve or receive a current time, $t_{current}$ from timer 628. Timer 628 can be a clock, a calendar, etc. ROM database 624 can store a datapoint including the range of motion angle $\theta_{ROM}$, the fully flexed angle $\theta_{flexed}$, the fully extended angle $\theta_{extend}$, and the corresponding time $t$ at which the angular measurements are obtained/recorded. ROM database 624 can also receive the determined/identified positions of locators 20 from locator position manager 620 and store the positions of locators 20 that are used to calculate the angular values and the range of motion angle.

ROM database 624 can store any of the received angular values, the positions of locators 20, and the time at which the measurement is recorded or obtained as a table, a chart, a matrix, vectors, time series data, etc. In some embodiments, ROM database 624 stores the angular values (e.g., $\theta_{ROM}$, $\theta_{extend}$, $\theta_{flexed}$, etc.), the positions of locators 20 used to determine the angular values, and the time at which the angular values are recorded/obtained in a CSV file. ROM database 624 can also be configured to receive the image data used to obtain/calculate the range of motion angle from imaging device 614 and/or locator position manager 620 and store the image data (e.g., the fully flexed and the fully extended image data files) with the corresponding time at which the image data is recorded/obtained.

In some embodiments, ROM database 624 is configured to provide timer 628 and/or display manager 630 with a time $t_{prev}$ at which the previous angular values (e.g., the range of motion angle) was recorded. Timer 628 and/or display manager 630 can use a current time (e.g., a current date, a current time of day, etc.,) to determine an amount of elapsed time since the previous range of motion was obtained. Timer 628 and/or display manager 630 can be configured to compare the amount of elapsed time to a threshold value $\Delta t_{ROM}$ (e.g., 24 hours, 48 hours, 1 week, etc.). The threshold value can be a frequency of how often the patient should record the range of motion angle. For example, $\Delta t_{ROM}$ can be 24 hours (indicating that the range of motion angle of joint 12 should be recorded daily), 48 hours (indicating that the range of motion angle of joint 12 should be recorded every other day), etc. In some embodiments, $\Delta t_{ROM}$ is a predetermined threshold value. $\Delta t_{ROM}$ can be a value set by a clinician or a medical professional. For example, if the clinician desires the patient to record the range of motion angle of joint 12 daily, the clinician can set $\Delta t_{ROM}$ to a 24 hour period. The clinician can remotely set or adjust (e.g., increase or decrease) the threshold value $\Delta t_{ROM}$.

The threshold value $\Delta t_{ROM}$ can be a value that is set at a beginning of NPWT and remains the same over an entire duration of a NPWT therapy time (e.g., a month, a week, two weeks, etc.). In some embodiments, the threshold value $\Delta t_{ROM}$ changes according to a schedule as the NPWT progresses. For example, the threshold value $\Delta t_{ROM}$ may be a smaller value (e.g., 24 hours) over a first time interval of the NPWT therapy time (e.g., the first week), and a larger value (e.g., 48 hours) over a second time interval of the NPWT therapy time. The clinician can set the schedule of $\Delta t_{ROM}$ at a beginning of NPWT. The clinician can remotely set, adjust, or change the schedule of $\Delta t_{ROM}$ (e.g., with clinician device 612 that is configured to wirelessly communicate with smartphone 100).

Display manager 630 and/or timer 628 compare the amount of elapsed time since the previously recorded range of motion angle to the threshold value $\Delta t_{ROM}$ to

determine if the patient should record the range of motion angle $\theta_{ROM}$. If the amount of time elapsed since the previously recorded range of motion angle is greater than or equal to the threshold value $\Delta t_{ROM}$, display manager 630 can operate touchscreen 102 to provide a notification, a message, a reminder, a pop-up, etc., to the patient. The notification can remind the patient that it is time to record the range of motion angle of joint 12 and prompt the patient to launch the application. In some embodiments, the notification or reminder includes a value of amount of elapsed time since the previously recorded range of motion angle.

In some embodiments, display manager 630 is configured to notify or prompt the patient to record the range of motion angle before the time since the last recorded range of motion angle is greater than or equal to the threshold value $\Delta t_{ROM}$. For example, display manager 630 can pre-emptively remind, notify, prompt, etc., the patient to launch the application to record the range of motion angle to ensure that the patient does not forget to record the range of motion angle.

Smartphone 100 can launch the application in response to receiving a user input via touchscreen 102. When the application is launched, the application can transition into a flex mode, and an extend mode. When in the flex mode, display manager 630 can provide a message to the patient through touchscreen 102 (or any other display device) to record an image with joint 12 fully flexed. Likewise, when in the extend mode, display manager 630 can provide a message to the patient through touchscreen 102 to record an image with joint 12 fully extended. The images can be captured by smartphone 100 and provided to locator position manager 620 and calibration manager 618 in response to a user input via touchscreen 102 (e.g., in response to the user pressing a button on touchscreen 102).

Referring still to FIG. 6, memory 606 includes a reporting manager 626, according to some embodiments. Reporting manager 626 can be configured to retrieve currently recorded/written/stored data from ROM database 624, and/or previously recorded/written/stored data from ROM database 624. Reporting manager 626 can generate a report and operate touchscreen 102 to display the report to the patient. The report can include any of range of motion improvement information, graphs showing the range of motion angle values over time, remaining NPWT therapy time, alerts, number of missed range of motion angle values, range of motion angle recording schedules, tabular information of the range of motion angle over time, etc.

In some embodiments, reporting manager 626 is configured to operate touchscreen 102 to display the report in response to receiving a request from touchscreen 102 that the patient desires to see the report. The report provided to the patient can be generated based on user inputs. For example, the patient can indicate that the report should include a time series graph, tabular information, percent improvements in the range of motion angle, etc.

In some embodiments, reporting manager 626 is configured to automatically provide the report to the patient via touchscreen 102 in response to the range of motion angle being recorded. For example, after the patient launches the application, captures images, and the range of motion angle is determined, reporting manager 626 may operate touchscreen 102 to display a current value of the range of motion angle, a percent improvement since the previously recorded range of motion angle, a total percent improvement since the first recorded range of motion angle, etc.

For example, reporting manager 626 can identify a first recorded/obtained range of motion angle $\theta_{ROM,1}$, and compare $\theta_{ROM,1}$ to a current range of motion angle $\theta_{ROM,current}$. Reporting manager 626 can determine a difference, a percent change, an increase, etc., between $\theta_{ROM,1}$ and $\theta_{ROM,current}$ and display the difference, the percent change, the increase, etc., to the patient via touchscreen 102. In some embodiments, reporting manager 626 determines a difference, a percent change, an increase, etc., between the current range of motion angle $\theta_{ROM,current}$ and a previously obtained range of motion angle, and displays the difference, the percent change, the increase, etc., to the patient via touchscreen 102.

In some embodiments, reporting manager 626 generates and provides the reports to a remote device, shown as clinician device 612. Clinician device 612 can be a remote device that is communicably connected with smartphone 100 via communications interface 608. Clinician device 612 and smartphone 100 can be configured to communicate via the Internet, a network, a cellular network, etc. Clinician device 612 and smartphone 100 can be wirelessly communicably coupled. Clinician device 612 can launch a messaging application, a chat application, send an email, send an SMS, etc., to smartphone 100. A clinician can provide real-time feedback and communication to the patient via clinician device 612 and smartphone 100. In some embodiments, the clinician device can initiate or launch the messaging or chat application in response to receiving a progress report from reporting manager 626. Advantageously, this allows the clinician to remotely monitor range of motion and healing progress without requiring the patient to visit the clinic. Clinician device 612 can access the patient's calendar and schedule a clinic appointment.

A clinician can send a request from clinician device 612 to smartphone 100 to obtain the report from smartphone 100. Reporting manager 626 can generate and provide the reports to clinician device 612 every time a new range of motion angle value is recorded. In some embodiments, reporting manager 626 provides the reports to clinician device 612 in response to receiving the request from clinician device 612. The reports provided to clinician device 612 by reporting manager 626 can include image data associated with any of the range of motion angles. In this way, a clinician can remotely monitor healing progress, progress in the range of motion of joint 12, etc. The clinician can receive the reports periodically (e.g., automatically every day, every week, in response to a new range of motion angle measurement, etc.) or can receive the reports on a request basis. This facilitates allowing a clinician to remotely monitor and check up on healing progress of joint 12.

Referring still to FIG. 6, remote device 610 is shown communicably connected with smartphone 100. In some embodiments, remote device 610 is communicably connected with smartphone 100 via communications interface 608. Remote device 610 can be any computer, server, network device, etc., configured to upload, install, etc., any of the functionality described herein to smartphone 100. For example, remote device 610 can install the application on smartphone 100 for performing any of the functionality described herein. Remote device 610 can install the application on smartphone 100 in response to receiving a request from smartphone 100 to install the application. For example, the patient can navigate to an application store, a website, etc., and install the application. Remote device 610 then provides installation packages, programs, etc., and configures processing circuit 602 to perform any of the functionality described herein. Remote device 610 can install any of the instructions, programs, functions, etc., necessary to perform the functionality described herein on smartphone 100 locally. Remote device 610 can configure smartphone 100 to communicably connect with remote device 610 to send image data so that any of the functionality of smartphone 100 described herein can be performed remotely.

Remote device 610 can perform any of the functionality of smartphone 100 to measure or obtain the range of motion angle values. In some embodiments, remote device 610 is configured to perform any of the functionality of calibration manager 618, locator position manager 620, ROM manager 622, timer 628, display manager 630, ROM database 624, reporting manager 626, etc. Remote device 610 can receive the image data from imaging device 614 of smartphone 100, perform the processes described herein remotely, and provide smartphone 100 with the obtained angular values or positions of locators 20.

Referring still to FIG. 6, smartphone 100 includes a calibration manager 618, according to some embodiments. Calibration manager 618 is configured to analyze the image data or use an orientation of smartphone 100 to determine calibrate the range of motion angle. Calibration manager 618 can receive the flexed image data and/or the extended image data from imaging device 614. Calibration manager 618 can also receive an orientation value from a gyroscope, an accelerometer, a goniometer, etc., shown as orientation sensor 616. Calibration manager 618 can determine an orientation of smartphone 100 relative to the patient's limb. Calibration manager 618 can determine angular offset amounts for the range of motion angle to account for the orientation of smartphone 100 relative to the patient's limb.

Calibration manager 618 can use any image analysis techniques described herein to determine the orientation of smartphone 100 relative to the patient's limb, or can use the orientation of smartphone 100 recorded by orientation sensor 616, or some combination of both. In some embodiments, calibration manager 618 communicates with locator position manager 620. Calibration manager 618 can receive the locator positions from locator position manager 620 and identify shape, skew, size, etc., of locators 20 on the image to determine orientation of smartphone 100 relative to the patient's limb.

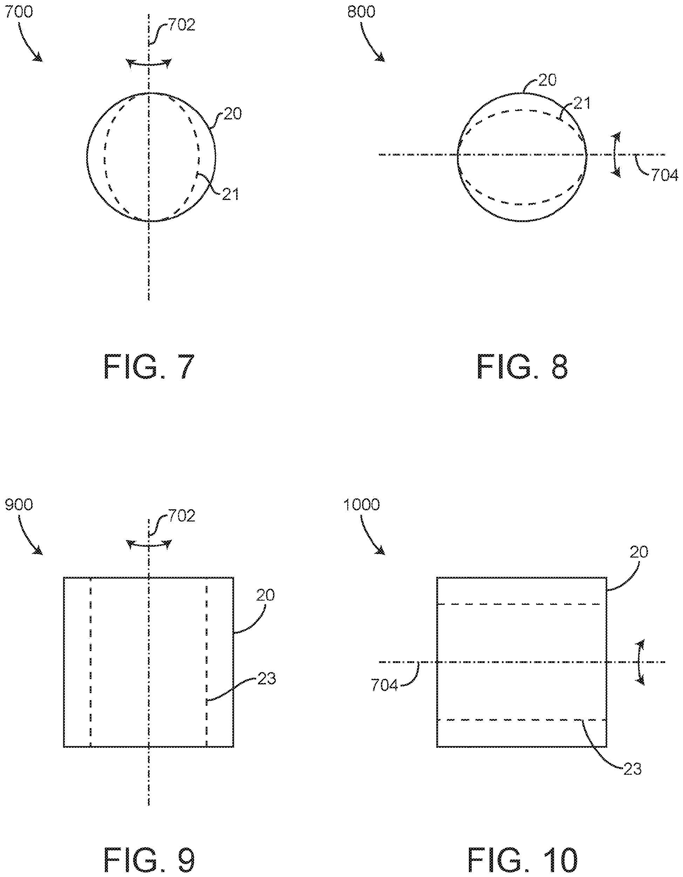

Referring now to FIGS. 7-10, diagrams 700, 800, 900, and 1000 show how calibration manager 618 determines orientation of smartphone 100 relative to the patient's limb by analyzing the shape of locators 20. Locators 20 have a predefined shape (e.g., a circle, a pentagon, a square, a star, a triangle, etc.). Calibration manager 618 can identify a shape of locators 20 based on the color of locators 20 with respect to background color, the contrast between locators 20 and the background, etc. Calibration manager 618 compares the shape of locators 20 to the predefined, known, shape of locators 20 to determine the orientation of smartphone 100 relative to the patient's limb. Calibration manager 618 uses the orientation of smartphone 100 relative to the patient's limb to determine an adjustment to any of the angular values (e.g., an adjustment to $\theta_{ROM}$, an adjustment to $\theta_{flexed}$, an adjustment to $\theta_{extend}$, etc.).

Referring particularly to FIGS. 7 and 8, locators 20 may have a circular shape. If locators 20 are rotated about a vertical axis 702 due to the orientation of smartphone 100 relative to the patient's limb, locators 20 can have the shape of an ellipse 21 as shown in FIG. 7. Likewise, if locators 20 are skewed or rotated about a horizontal axis 704 due to the orientation of smartphone 100 relative to the patient's limb, locators 20 can have the shape of ellipse 21 as shown in FIG. 8. In this way, the shape of locators 20 is related to the orientation of smartphone 100 relative to the patient's limb.

Calibration manager 618 can analyze the image to determine a shape of locators 20. Calibration manager 618 can compare the shape of locators 20 as shown in the image to the known shape of locators 20 when smartphone 100 is perpendicular to the patient's limb. In some embodiments, calibration manager 618 is configured to determine focal points of locators 20. Calibration manager 618 can determine linear eccentricity of the shape of locators 20 as captured in the image. Depending on the ellipticality of locators 20 in the captured image, calibration manager 618 can determine an orientation of smartphone 100 relative to the patient's limb about either vertical axis 702 or about horizontal axis 704, or about both axes 702 and 704.

Referring now to FIGS. 9 and 10, locators 20 can have the shape of a square, according to some embodiments. Locators 20 may skew about vertical axis 702 due to the orientation of smartphone 100 relative to the patient's limb. If locators 20 are skewed about vertical axis 702, locators 20 can have the appearance of a rectangle 23 as shown in FIG. 9. Likewise, locators 20 can be skewed about horizontal axis 704 due to the orientation of smartphone 100 relative to the patient's limb. If locators 20 are skewed about horizontal axis 704, locators 209 can have the appearance of rectangle 23 as shown in FIG. 9. Calibration manager 618 can be configured to compare the shape of locators 20 as they appear in the image to the known shape of locators 20 (e.g., a square). Calibration manager 618 can determine orientation of smartphone 100 relative to the patient's limb based on the deviation between the shape of locators 20 in the captured image and the known shape of locators 20. Calibration manager 618 can use the deviation or difference between the shape of locators 20 in the captured image and the known shape of locators 20 to determine orientation of smartphone 100 relative to the patient's limb about one or more axes.

In some embodiments, calibration manager 618 determines the orientation of smartphone 100 relative to the patient's limb, and/or the distance between smartphone 100 and the patient's limb based on initial images captured by smartphone 100. The initial images captured by smartphone 100 may be captured by a clinician. For example, a clinician can align smartphone 100 such that it is substantially perpendicular to the patient's limb and capture fully flexed and fully extended images. Smartphone 100 can then use any of the processes, techniques, functionality, etc., described in greater detail above to determine the range of motion angle $\theta_{ROM}$ for the initial images.

Calibration manager 618 can store the initial images and compare subsequently captured images to the initial images to determine orientation of smartphone 100 relative to the patient's limb, and/or distance between smartphone 100 and the patient's limb. In some embodiments, the initial images are captured by the clinician in a controlled environment. For example, the clinician can hold smartphone 100 a predetermined distance from the patient's limb and at an orientation such that smartphone 100 is substantially perpendicular to the patient's limb. For example, the predetermined distance may be about 61 cm (2 feet), 91 cm (3 feet), 76 cm (2.5 feet), etc. Calibration manager 618 may use the positions, shapes, distances, etc., of locators 20 in the initial images as baseline values. Calibration manager 618 can determine similar values for subsequently captured images and compare the values of the subsequently captured images to the baseline values to determine distance between smartphone 100 and the patient's limb, in addition to the orientation of smartphone 100 relative to the patient's limb.

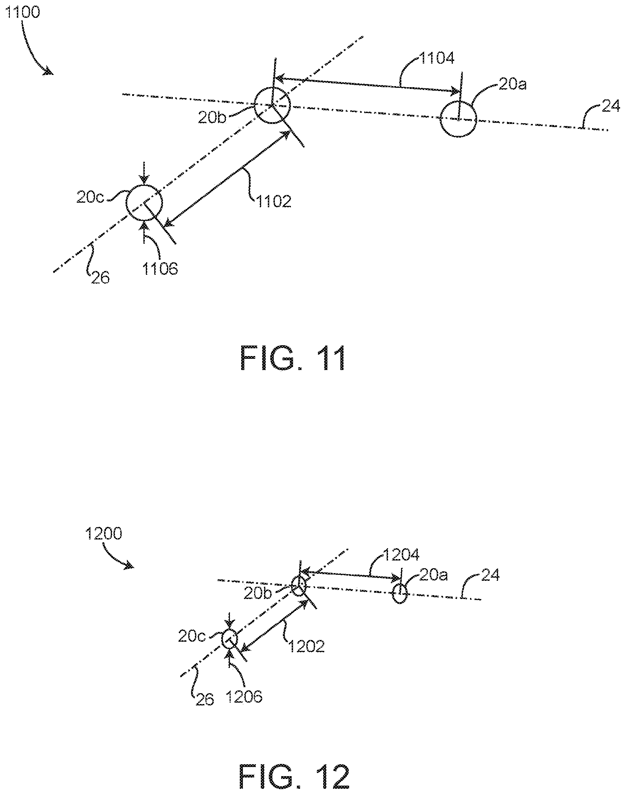

Referring to FIG. 11, diagram 1100 shows locators 20 and the values of the initial image captured by smartphone 100. Calibration manager 618 can determine a dimension 1106 of locators 20. For example, if locators 20 are circles, dimension 1106 can be a diameter, radius, area, etc., of locators 20. In some embodiments, dimension 1106 is an outer distance of locators 20 (e.g., a height of a rectangle, a distance between outer peripheries of locator 20 that extends through a center of locator 20, etc.).

Calibration manager 618 can also determine a distance 1104 between locators 20a and 20b, and a distance 1102 between locators 20b and 20c (assuming three locators 20 are used). In some embodiments, if four locators 20 are used, calibration manager 618 determines a distance between locators 20a and 20b, and a distance between locators 20b and 20c. Calibration manager 618 can use any imaging techniques similar to locator position manager 620 to determine distances between locators 20. Calibration manager 618 can use the positions of locators 20 as determined by locator position manager 620 to determine distances between locators 20.

Calibration manager 618 can also identify a shape of locators 20 based on the initial image(s). For example, calibration manager 618 can determine that the shape of locators 20 is a circle, a square, a star, etc.

Referring now to FIG. 12, diagram 1200 shows locators 20 and various values of an image captured in an uncontrolled environment by smartphone 100. For example, diagram 1200 can represent an image captured by the patient, where the distance between smartphone 100 and the patient's limb is different than the initial image. Additionally, diagram 1200 represents an image captured by the patient when the orientation of smartphone 100 relative to the patient's limb is non-perpendicular.

Calibration manager 618 can determine dimension 1206 (e.g., diameter, size, etc.) of locators 20 and compare dimension 1206 to dimension 1106. In some embodiments, calibration manager 618 determines a distance between smartphone 100 and the patient's limb for the image represented by diagram 1200 based on dimension 1206 of locators 20. For example, if locators 20 are circles, dimension 1206 can be a diameter, $d$. Calibration manager 618 can use a predetermined or predefined relationship and the value of $d$ to determine the distance between smartphone 100 and the patient's limb. The diameter d of locators 20 may decrease with increased distance between smartphone 100 and the patient's limb, while the diameter d of locators 20 may increase with decreased distance between smartphone 100 and the patient's limb. In this way, the diameter d of locators 20 can be used by calibration manager 618 with a relationship to determine the distance between smartphone 100 and the patient's limb.

Calibration manager 618 can similarly compare distance 1202 (between locator 20b and locator 20c) to distance 1102 to determine distance between smartphone 100 and the patient's limb. Distance 1202 and/or distance 1204 can have a relationship to the distance between smartphone 100 and the patient's limb similar to the relationship between the diameter d of locators 20 and the distance between smartphone 100 and the patient's limb (e.g., increased distance 1202 or increased distance 1204 corresponds to decrease distance between smartphone 100 and the patients limb, and vice versa). In this way, calibration manager 618 can use distance 1202 and/or distance 1204 to determine the distance between smartphone 100 and the patient's limb.

Calibration manager 618 can also identify changes or deviations in the shape of locators 20 as compared to the shape of locators 20 in the initial image. For example, locators 20 as shown in FIG. 12 are skewed about a vertical axis (not shown). Calibration manager 618 can determine a degree of skew, stretch, deformation, etc., of the shape of locators 20 relative to the shape of locators 20 in the initial image. In some embodiments, calibration manager 618 determines the degree of skew, stretch, deformation, etc., of the shape of locators 20 in multiple directions (e.g., in a horizontal direction and a vertical direction). The distance between smartphone 100 and the patient's limb may be referred to as r. The orientation of smartphone 100 relative to the patient's limb can include an azimuth angle $\phi_{az}$ and an elevation angle $\phi_{el}$. Calibration manager 618 can compare the subsequently captured images (or any values, properties, shape of locators 20, distance between locators 20, size of locators 20, etc.) to the initial captured image to determine the distance r, the azimuth angle $\phi_{az}$ and the elevation angle $\phi_{el}$.

Calibration manager 618 can use the orientation of smartphone 100 relative to the patient's limb to determine angular offset amounts or adjustments for $\theta_{extend}$, $\theta_{flex}$, and $\theta_{ROM}$ to account for the orientation of smartphone 100 relative to the patient's limb. In some embodiments, calibration manager 618 calculates the distance between smartphone 100 and the patient's limb (e.g., r) and/or the orientation of smartphone 100 relative to the patient's limb (e.g., the azimuth angle $\phi_{az}$ and the elevation angle $\phi_{el}$) in real-time and notifies the patient when smartphone 100 is properly aligned with the patient's limb. Calibration manager 618 can operate imaging device 614 to capture image data (e.g., take a picture) when smartphone 100 is properly oriented relative to the patient's limb (e.g., when $\phi_{az}$ and $\phi_{el}$ are substantially equal to zero, or desired values).

Calibration manager 618 can also record the orientation of smartphone 100 when the initial image is captured. In some embodiments, calibration manager 618 receives the orientation of smartphone 100 from orientation sensor 616. Calibration manager 618 can compare the orientation of smartphone 100 for later captured images to the orientation of smartphone 100 for the initial captured image to determine offsets or adjustments for $\theta_{extend}$, $\theta_{flex}$, and $\theta_{ROM}$ to account for the orientation of smartphone 100 relative to the patient's limb.

Calibration manager 618 can use the distance between smartphone 100 and the patient's limb (e.g., $r$), and/or the orientation of smartphone 100 relative to the patient's limb (e.g., $\phi_{az}$ and $\phi_{el}$) to determine offset or adjustment amounts $\theta_{extend,adj}$, $\theta_{flex,adj}$, and $\theta_{ROM,adj}$. For example, calibration manager 618 can use a predetermined function, relationship, equation, etc., to determine $\theta_{extend,adj}$, $\theta_{flex,adj}$, and $\theta_{ROM,adj}$ based on the distance between smartphone 100 and the patient's limb (e.g., $r$) and/or the orientation of smartphone 100 relative to the patient's limb (e.g., $\phi_{az}$ and $\phi_{eil}$). In some embodiments, calibration manager 618 provides the offset or adjustment amounts $\theta_{extend,adj}$, $\theta_{flex,adj}$, and $\theta_{ROM,adj}$ to ROM manager 622. ROM manager 622 can use the offset/adjustment amounts $\theta_{extend,adj}$, $\theta_{flex,adj}$, and $\theta_{ROM,adj}$ to adjust (e.g., increase, decrease, etc.) the values of $\theta_{extend}$, $\theta_{flexed}$, and $\theta_{ROM}$. For example, ROM manager 622 may add $\theta_{extend,adj}$ to $\theta_{extend}$ or subtract $\theta_{extend,adj}$ from $\theta_{extend}$ to account for orientation of smartphone 100 relative to the patient's limb.

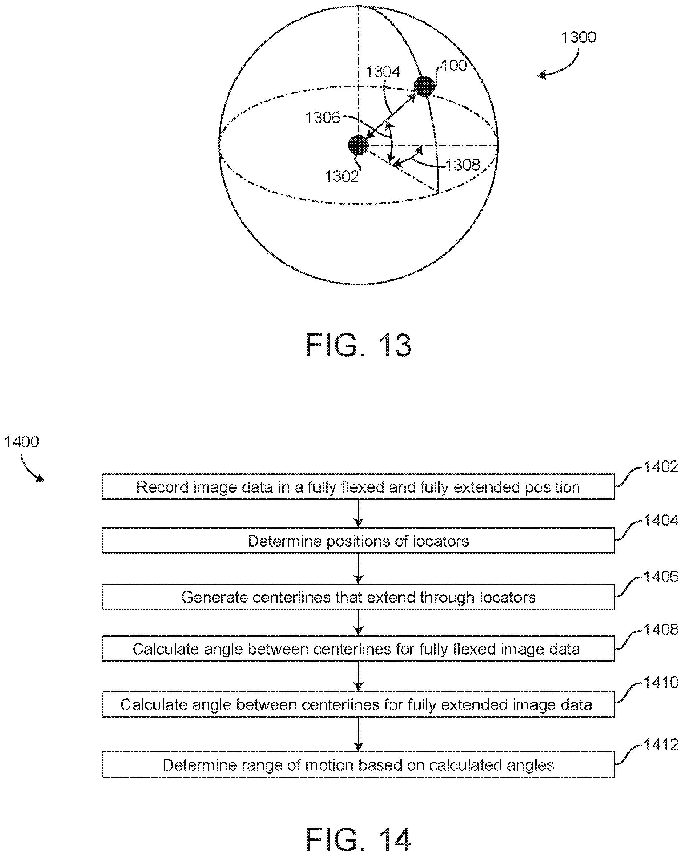

Referring now to FIG. 13, diagram 1300 illustrates relative orientation between smartphone 100 and a point of interest 1302. Point of interest 1302 can be the patient's limb. Calibration manager 618 is configured to use any of the techniques described in greater detail above to determine distance 1304 (i.e., r, the distance between smartphone 100 and the patient's limb), angle 1308 (i.e., the azimuth angle $\phi_{az}$), and angle 1306 (i.e., the elevation angle $\phi_{el}$). In some embodiments, calibration manager 618 is also configured to determine a local orientation of smartphone 100.

Referring again to FIG. 6, ROM manager 622 adjusts or offsets any of the angles $\theta_{extend}$, $\theta_{flex}$, and $\theta_{ROM}$ and provides the angles $\theta_{extend}$, $\theta_{flex}$, and $\theta_{ROM}$ to ROM database 624. In this way, the application can account for orientation of smartphone 100 relative to the patient's limb.

Advantageously, the application can be installed on smartphone 100 by a clinician and/or by a patient. In some embodiments, the application is installed and set up by a clinician. The clinician can set various initial parameters (e.g., frequency of range of motion measurements, when reports should be provided to the patient, when reports should be provided to clinician device 612, what information is displayed to the patient

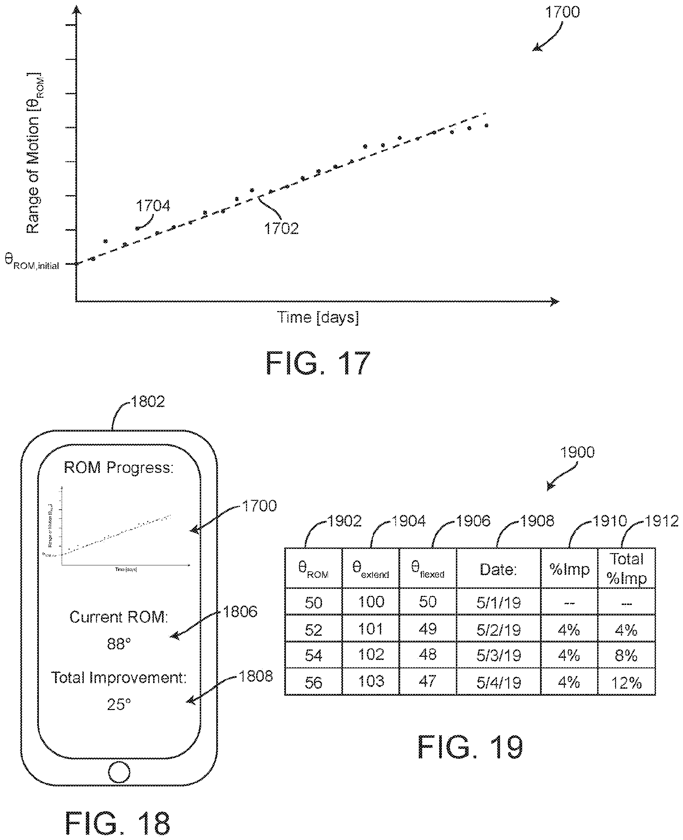

Referring now to FIG. 17, reporting manager 626 can generate a graph 1700 based on the recorded/stored data in ROM database 624. Reporting manager 626 can generate graph 1700 that shows the range of motion angle $\theta_{ROM}$ (the Y-axis) over time (the X-axis). In some embodiments, reporting manager 626 retrieves time series data from ROM database 624 and plots the range of motion angle $\theta_{ROM}$ against the corresponding dates or times at which the range of motion angles $\theta_{ROM}$ were recorded/measured. Reporting manager 626 may plot scatter data 1704 retrieved from ROM database 624. Reporting manager 626 can perform a linear regression to generate a trendline 1702 that shows the overall trend of the healing process. Reporting manager 626 may be configured to generate graphs similar to graph 1700 for any of percent improvement in the range of motion angle $\theta_{ROM}$, the flexed angle $\theta_{flexed}$, and the extended angle

$\theta_{extend}$. Reporting manager 626 can display any of the generated graphs to the patient via touchscreen 102. Reporting manager 626 can be configured to operate smartphone 100 to wirelessly provide the generated graphs to clinician device 612. Reporting manager 626 can generate the graphs in response to receiving a request from clinician device 612. Reporting manager 626 can also provide the generated graphs to clinician device 612 in response to receiving the request.

Referring now to FIG. 18, reporting manager 626 and/or display manager 630 can operate a mobile device, a smartphone, a tablet, a computer, a stationary computer, a desktop computer, a display screen, a touchscreen, etc., shown as user device 1802 to display graph 1700 to a patient or a clinician. User device 1802 can be the patient's smartphone 100. In some embodiments, user device 1802 is clinician device 612. For example, the patient's smartphone 100 and/or clinician device 612 can include a display screen configured to display information (e.g., graph 1700, tabular information, range of motion angle information, etc.).

Reporting manager 626 and/or display manager 630 can also operate user device 1802 to display a currently calculated or a previously calculated (e.g., a most recent) range of motion angle notification 1806. Reporting manager 626 and/or display manager 630 can also operate user device 1802 to display a notification 1808 including a percent improvement since a previously recorded range of motion angle, a total percent improvement since an initially recorded range of motion angle, a total improvement (e.g., in degrees) since the previously recorded range of motion data, a total improvement (e.g., in degrees) since the initially recorded range of motion data, etc. Display manager 630 and/or reporting manager 626 can also display current or most recently calculated flexed angle values $\theta_{flexed}$, current or most recently calculated extension angle $\theta_{extend}$, percent improvements (e.g., since previously recorded values or since initially recorded values) of $\theta_{flexed}$ and/or $\theta_{extend}$, total improvements (e.g., an angular improvement since previously recorded values or since initially recorded values) of $\theta_{flexect}$ and/or $\theta_{extend}$, etc. Display manager 630 and/or reporting manager 626 can also operate user device 1802 to display historical data (e.g., in tabular form) of any of the information stored in ROM database 624 (e.g., $\theta_{ROM}$, $\theta_{flexed}$, $\theta_{extend}$, dates/times of recorded measurements, etc.).

Referring now to FIG. 19, a table 1900 shows information that can be display to the patient (e.g., via smartphone 100) or to a clinician (e.g., via clinician device 612) is shown. Table 1900 includes a range of motion column 1902, an extension column 1904, a flexion column 1906, a date column 1908, a percent improvement column 1910, and a total percent improvement column 1912. In some embodiments, table 1900 includes range of motion angle $\theta_{ROM}$ values in rows of column 1902. Table 1900 can include extension angle $\theta_{extend}$ values in rows of column 1904. Table 1900 can flexion angles

$\theta_{flexed}$ values in rows of column 1906. Table 1900 can include corresponding dates at which the image data used to determine the values of columns 1902-1906 and 1910-1912 was recorded in rows of column 1908. Table 1900 can include range of motion percent improvement since a most recently recorded/measured range of motion angle $\theta_{ROM}$ value. Table 1900 can include total range of motion percent improvement since an initially recorded/measured range of motion angle $\theta_{ROM,initial}$.

Table 1900 can be stored in ROM database 624 and retrieved by reporting manager 626. In some embodiments, table 1900 is displayed on or transmitted to clinician device 612. Table 1900 can be displayed to the patient via touchscreen 102. The values of columns 1902, 1904, and 1906 can be determined by ROM manager 622 based on positions of locators 20 and/or based on image data. The values of column 1908 can be recorded/captured by timer 628. The values of columns 1910 and 1912 can be determined by reporting manager 626.

Referring now to FIG. 14, a process 1400 for determining a range of motion of a joint based on image data is shown. Process 1400 includes steps 1402-1412, according to some embodiments. Process 1400 can be performed by a mobile application of a smartphone. Process 1400 can be performed locally by a processing circuit of a patient's smartphone. Process 1400 can be partially performed locally by the processing circuit of the patient's smartphone (e.g., step 1402 is performed locally) and partially performed remotely by another computer (e.g., steps 1404-1412 are performed by a remote server). Process 1400 can be performed to determine range of motion of a joint at various times over a time duration to track healing progress and improvements in the range of motion of the patient's joint.

Process 1400 includes recording image data in both a fully flexed and fully extended position (step 1402). Step 1402 includes providing a notification to the patient to extend the joint into the fully extended position and capture an image, and to flex the joint into the fully flexed position and capture an image. Step 1402 is performed by an imaging device. For example, step 1402 can be performed by imaging device 614 of smartphone 100.

Process 1400 includes determining positions of locators that are positioned about the joint (step 1404). The locators are positioned on both the upper and lower limbs of the jointed limb. Three locators are positioned on the joint, with the first locator being positioned on the upper limb, the second locator being positioned on the joint, and the third locator being positioned on the lower limb. In some embodiments, four locators are positioned on the limb, with a first set of two locators being positioned on the upper limb, and a second set of two locators being positioned on the lower limb. Step 1404 can include analyzing any of the recorded image data of the fully flexed and the fully extended joint. Step 1404 can include using an image processing technique (e.g., a neural network technique, an object detection technique, an edge detection technique, etc.) to determine the positions of the locators. The positions of the locators can be determined as Cartesian coordinates relative to an origin (e.g., an upper left corner of the image, a lower right corner of the image, a center of the image, a lower left corner of the image, etc.). Step 1404 can be performed by locator position manager 620 to determine the positions of locators 20 based on the recorded image data.

Process 1400 includes generating centerlines that extend through the determined positions of the locators (step 1406). In some embodiments, the centerlines are lines. The centerlines are centerlines 24 and 26. The centerlines are generated based on the determined positions of locators 20. The centerlines extend through a center of locators 20. Step 1406 can be performed by ROM manager 622.

Process 1400 includes calculating an angle between the centerlines for the fully flexed image data (step 1408) and the fully extended image data (step 1410). Steps 1408 and 1410 can be performed by ROM manager 622. Step 1408 can include determining $\theta_{flexed}$ and step 1410 can include determining $\theta_{extend}$. The angles can be determined using trigonometric identities, equations of the centerlines, the determined positions of the locators, etc.

Process 1400 includes determining a range of motion (i.e., $\theta_{ROM}$) based on the calculated/determined angles (step 1412). In some embodiments, the range of motion is an angular value. The range of motion may be a difference between the calculated angles. For example, the range of motion can be $\theta_{ROM} = \theta_{extend} - \theta_{flexed}$. Step 1412 can be performed by ROM manager 622.

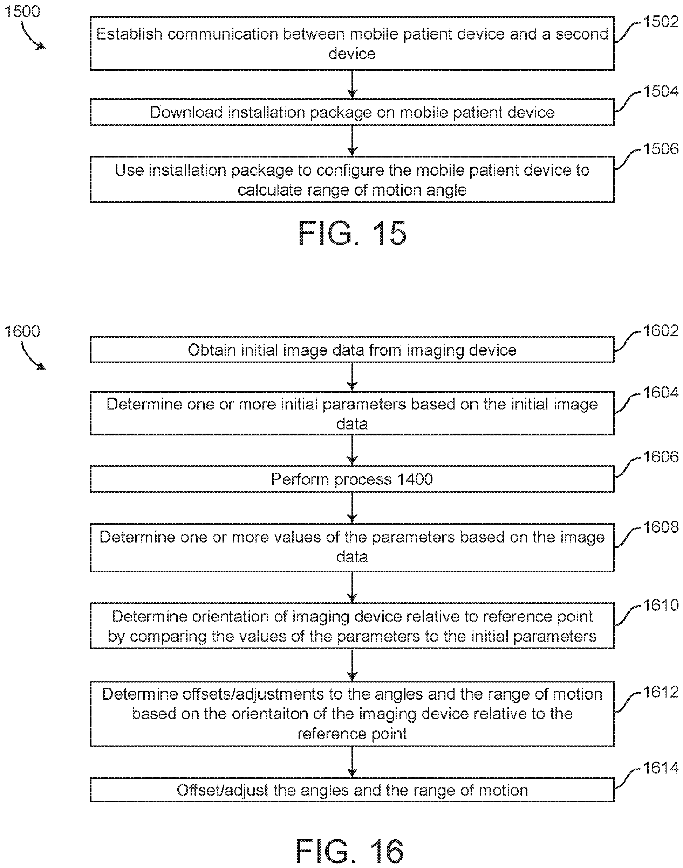

Referring now to FIG. 15, a process 1500 for configuring a device to perform any of the functionality, techniques, processes, programs, etc., described herein to calculate the range of motion angle $\theta_{ROM}$ and to track healing progress. Process 1500 includes steps 1502-1506. Process 1500 can be initiated by a patient or by a clinician. For example, a clinician may initiate process 1500 to set up the patient's smartphone 100. It should be understood that process 1500 can be performed for any personal computer device that has the required hardware (e.g., an imaging device, an accelerometer, etc.) for performing the processes described herein.

Process 1500 includes establishing communication between a patient's mobile device (e.g., smartphone 100) and a second device (e.g., remote device 610) (step 1502), according to some embodiments. The communication between the patient's mobile device and the second device may be a wireless connection. The communication between the patient's mobile device and the second device may be a wired connection. For example, smartphone 100 can wirelessly communicably connect with the second device, which can be remotely positioned. The patient's mobile device and the second device may be wirelessly or wiredly connected in a clinic by a clinician. Step 1502 can be performed by smartphone 100, communications interface 608, a clinician, the patient, etc.

Process 1500 includes downloading or transferring an installation package onto the patient's mobile device (step 1504), according to some embodiments. The installation package can be transferred to the patient's mobile device from the second device. The installation package can be any of an .apk file, a .pkg file, etc., or any other package file or installation package file. Step 1504 can be performed by smartphone 100.

Process 1500 includes using the installation package to configure the patient's mobile device to calculate the range of motion angle $\theta_{ROM}$ and to perform any of the other processes, functionality, etc., described herein (step 1506), according to some embodiments. Step 1506 can be performed by smartphone 100 using the installation package received from the second device (e.g., received from clinician device 612 and/or remote device 610).

Referring now to FIG. 16, a process 1600 for offsetting/adjusting any of the flexion angle $\theta_{flexed}$, the extension angle $\theta_{extend}$, and the range of motion angle $\theta_{ROM}$ is shown, according to some embodiments. Process 1600 includes steps 1602-1614. Process 1600 can be performed after or in conjunction with process 1400. Process 1600 can be performed to adjust (e.g., increase or decrease) any of the angles determined in process 1400 to account for relative orientation between the patient's smartphone when the image was captured, and the patient's joint. Process 1600 can be performed after process 1500.

Process 1600 includes obtaining initial image data from an imaging device (step 1602), according to some embodiments. Step 1602 can be the same as or similar to step 1402 of process 1400. The initial image data can be captured by a clinician or a patient. For example, a clinician can use the patient's smartphone or mobile device to capture the initial image data. The clinician may align the patient's smartphone such that the smartphone is substantially perpendicular to the patient's joint or perpendicular to locators 20. The clinician can also capture the initial image data at a predetermined distance from the patient's joint. The initial image data can be recorded at $\phi_{az} \approx 0$ and $\phi_{el} \approx 0$ such that locators 20 are substantially perpendicular to a line of sight of imaging device 614 of the patient's smartphone 100.

Process 1600 includes determining one or more initial parameters based on the initial image data (step 1604), according to some embodiments. Step 1604 can include analyzing the initial image/image data to determine relative distances between locators 20, identify an initial shape, size, skew, etc., of locators 20, etc. Step 1604 can include receiving or capturing an initial orientation of smartphone 100 from orientation sensor 616. Step 1604 may be performed by calibration manager 618.

Process 1600 includes performing process 1400 (step 1606), according to some embodiments.

Process 1400 can be performed at regularly spaced intervals according to a schedule. Process 1400 can be performed to obtain image data at various points in time along the healing process.

Process 1600 includes determining one or more values of the parameters based on the image data obtained in step 1606 (step 1608), according to some embodiments. Step 1608 can be performed by calibration manager 618. Calibration manager 618 can determine any of the parameters of step 1604 for the newly obtained images. For example, calibration manager 618 can analyze the newly obtained images/image data to determine relative distance between locators 20, shape, size, skew, etc., of locators 20, etc.

Process 1600 includes determining an orientation of the imaging device relative to a reference point (the patient's limb, locators 20, etc.) by comparing the values of the parameters of the newly obtained image to the initial parameters of the initial image (step 1610), according to some embodiments.

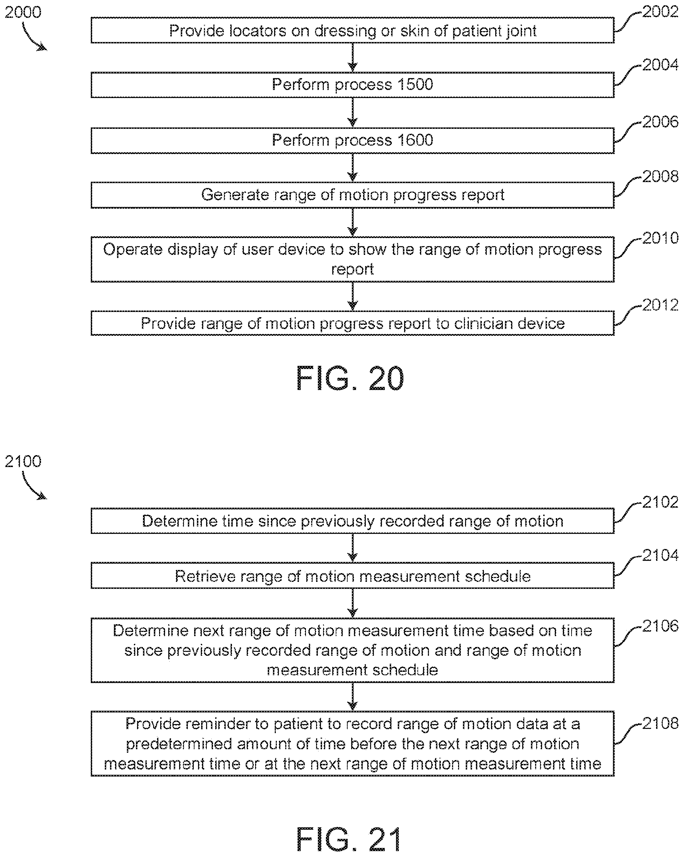

Referring now to FIG. 20, a process 2000 for monitoring range of motion or healing of a joint is shown. Process 2000 includes steps 2002-2012, according to some embodiments. Process 2000 can be performed with a NPWT system. Process 2000 can be performed at least partially in a clinical setting and at least partially in a patient's home. Process 2000 facilitates a clinician to remotely monitor healing progress of a patient's joint over time.

Process 2000 includes providing locators on a dressing or skin of a patient's joint (step 2002), according to some embodiments. Step 2002 can include adhering locators 20 to the patient's skin 32. Locators 20 can be adhered directly to the patient's skin or can be adhered to drape 18. Locators 20 can be printed on drape 18 by a drape manufacturer. Step 2002 can be performed by a clinician. For example, the clinician can adhere three or four (or more) locators 20 to the patient's jointed limb for tracking. Step 2002 can be performed periodically when dressing 36 is changed.

Process 2000 includes performing process 1500 to configure the patient's smartphone 100 to record and measure range of motion of the patient's joint (step 2004), according to some embodiments. Step 2004 can be initiated by a clinician in a clinical setting. Step 2004 can include setting various parameters such as measurement interval, measurement schedule, reminder schedules, etc., to ensure that the patient records range of motion when necessary.

Process 2000 includes performing process 1600 to obtain range of motion values of the patient's jointed limb (step 2006), according to some embodiments. Step 2006 can be performed multiple times over NPWT to obtain range of motion values as the patient's wound heals. Step 2006 can be initiated by a patient. Step 2006 can be initiated a first time by a clinician in a controlled environment to obtain baseline image data.

Process 2000 includes generating a range of motion progress report (step 2008), according to some embodiments. The range of motion progress report can include graphs, tabular information, historical information, analysis, etc., of the range of motion of the patient's jointed limb. Step 2008 can be performed by reporting manager 626. Step 2008 can include retrieving historical range of motion data from ROM database 624. The range of motion progress report can also include a currently calculated range of motion angle, percent improvements in the range of motion of the patient's joint, image data, etc.

Process 2000 includes operating a display of a user device to show the range of motion progress report (step 2010), according to some embodiments. Step 2010 can be performed by display manager 630. Display manager 630 can operate touchscreen 102 to display the generated range of motion progress report. Display manager 630 can operate touchscreen 102 of smartphone 100 to display any of the tabular information, the range of motion graphs, etc.

Process 2000 includes providing the range of motion progress report to a clinician device (step 2012), according to some embodiments. The range of motion progress report can be provided to clinician device 612. The range of motion progress report can be provided to clinician device 612 in response to smartphone 100 receiving a request from clinician device 612. The range of motion progress report can be provided to clinician device 612 in response to obtaining a new range of motion measurement of the patient's joint. The range of motion progress report can be provided to clinician device 612 periodically to that the clinician can monitor healing progress of the patient's wound. The range of motion progress report can include historical range of motion data, graphs, images used to calculate the range of motion, etc. Providing the range of motion progress report to clinician device 612 facilitates allowing a clinician to remotely monitor healing progress. The clinician can identify unexpected changes or problems with the healing progress. Process 2000 can include an additional step of receiving a notification from clinician device 612. For example, if the clinician determines, based on the received range of motion progress report, that the patient should come in to the clinic, the clinician can send a notification to the patient's smartphone 100 indicating that the patient should come in to the clinic. The clinician can launch a chat application and can send a message to the patient's smartphone.

Referring now to FIG. 21, a process 2100 for notifying a patient to record range of motion is shown, according to some embodiments. Process 2100 can include steps 2102-2108. Process 2100 can be performed periodically to determine if the patient should measure the range of motion of the patient's jointed limb.

Process 2100 includes determining an amount of time since a previously recorded range of motion (step 2102), according to some embodiments. Step 2102 can include determining an amount of elapsed time between a present time and a time at which the previous range of motion was recorded. The time interval can be in hours, days, minutes, etc. Step 2102 can be performed by timer 628 by comparing a current time value to a time at which the previously recorded range of motion was measured. The time at which the previously recorded range of motion was measured may be retrieved from ROM database 624.

Process 2100 includes retrieving a range of motion measurement schedule (step 2104), according to some embodiments. The range of motion measurement schedule can be retrieved from ROM database 624. The range of motion measurement schedule can be stored in timer 628. The range of motion measurement schedule can be predetermined or set at a beginning of NPWT by a clinician. The measurements of the range of motion can be scheduled at regular time intervals (e.g., every day, every week, etc.). Step 2104 may be performed by timer 628.

Process 2100 includes determining a next range of motion measurement time based on the amount of time since the previously recorded range of motion and the range of motion measurement schedule (step 2106), according to some embodiments. The next range of motion measurement time can be retrieved from the range of motion measurement schedule. Step 2106 can include determining an amount of time from a present/current time to the next range of motion measurement time. In some embodiment, step 2106 is performed by timer 628 and/or display manager 630.

Process 2100 includes providing a reminder to the patient to record the range of motion data at a predetermined amount of time before the next range of motion measurement time, or at the next range of motion measurement time (step 2108), according to some embodiments. A notification/reminder can be provided to the patient a predetermined amount of time before the next range of motion measurement time. For example, display manager 630 can operate touchscreen 102 to provide the patient with a reminder or notification that the next range of motion measurement should be recorded/captured within the next 12 hours, the next 24 hours, the next 5 hours, etc. Step 2108 can be performed when the current time is substantially equal to the next range of motion measurement time. Step 2108 can be performed by display manager 630 and/or timer 628. Process 2100 can be performed to ensure that the patient does not forget to capture/record range of motion angular values.

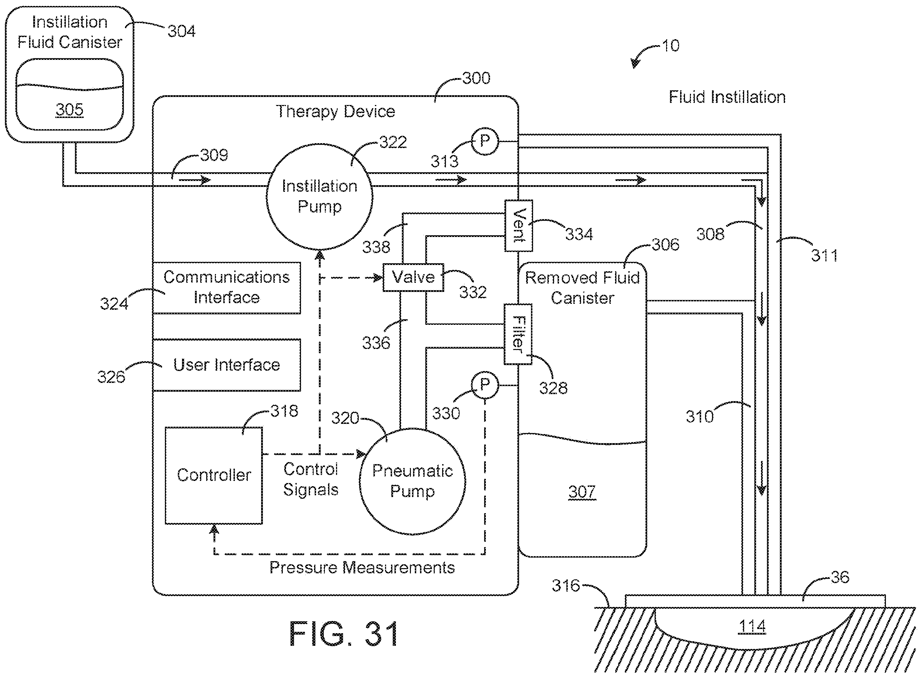

Referring now to FIG. 31, another process 3200 for performing negative pressure wound therapy and calculating the range of motion is shown, according to some embodiments. Process 3200 includes steps 3202-3212 and can be performed by any of the systems, controllers, etc., described herein. Process 3200 can be performed to apply negative pressure wound therapy to a wound, to calculate the range of motion of a jointed limb at which the wound is positioned, and to re-apply the negative pressure.

Process 3200 includes providing a dressing including a comfort layer, a manifold layer, and a drape (step 3202), according to some embodiments. The comfort layer can be a PREVENA™ layer. The comfort layer can be the wound-interface layer 128 (as described in greater detail below). The dressing can be dressing 36 and may be provided over or applied to a wound at a jointed limb.

Process 3200 includes providing one or more locators on the dressing (step 3204), according to some embodiments. The locators can be provided onto the drape layer (e.g., the drape 18, the drape layer 120). The locators can be provided onto an exterior surface of the drape layer or onto an interior surface of the drape layer if the drape layer is transparent or translucent. The locators can be printed, adhered, etc., or otherwise coupled with the drape layer such that the locators can be viewed on the dressing.

Process 3200 includes applying negative pressure to the wound at the dressing (step 3206), according to some embodiments. The negative pressure can be applied to the wound at the dressing by the therapy device 300. The therapy device 300 can fluidly couple with the dressing through a conduit that fluidly couples with an inner volume of the dressing.

Process 3200 includes relieving the applied negative pressure after a time duration (step 3208), according to some embodiments. The applied negative pressure can be relieved after a time duration (e.g., after the negative pressure is applied for some amount of time). Step 3208 may be performed by the therapy device 300 and/or the controller 318 of the therapy device 300.

Process 3200 includes performing process 2000 to calculate range of motion of the jointed limb (step 3210), according to some embodiments. Step 3210 can include performing any of the processes 1400, 1500, 1600, 2000, and/or 2100. Step 3210 is performed to calculate the range of motion using the locators on the dressing. The negative pressure may be relieved prior to performing step 3210.

Process 3200 includes re-applying the negative pressure to the wound at the dressing (step 3212), according to some embodiments. The negative pressure can be re-applied after step 3210 is performed. For example, the negative pressure can be re-applied to the wound at the dressing in response to calculating the range of motion of the jointed limb. Step 3212 can be performed by the controller 318.

Wound Dressing

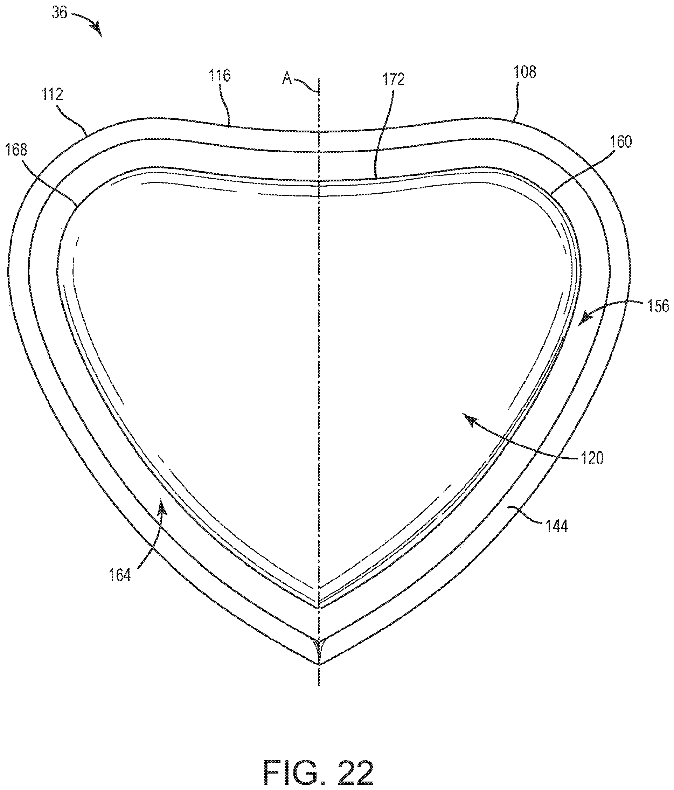

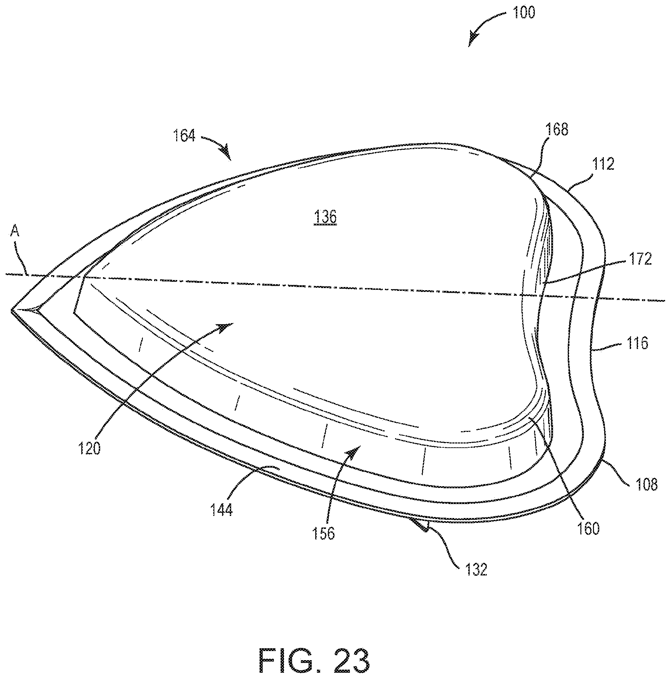

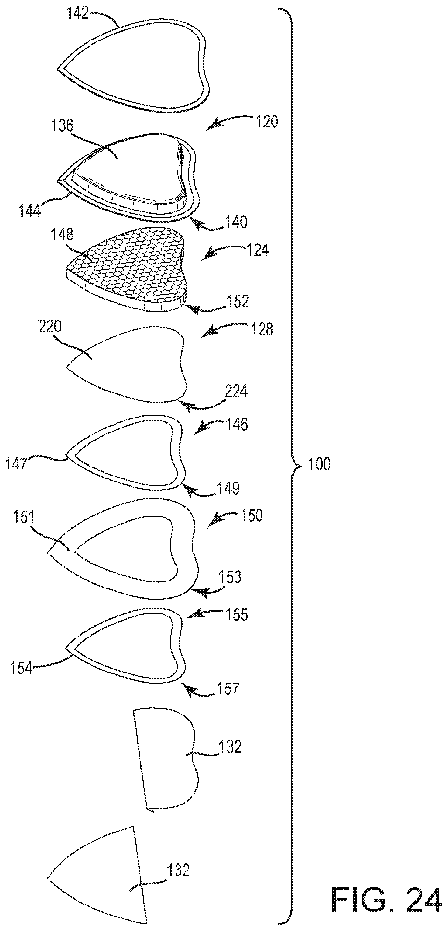

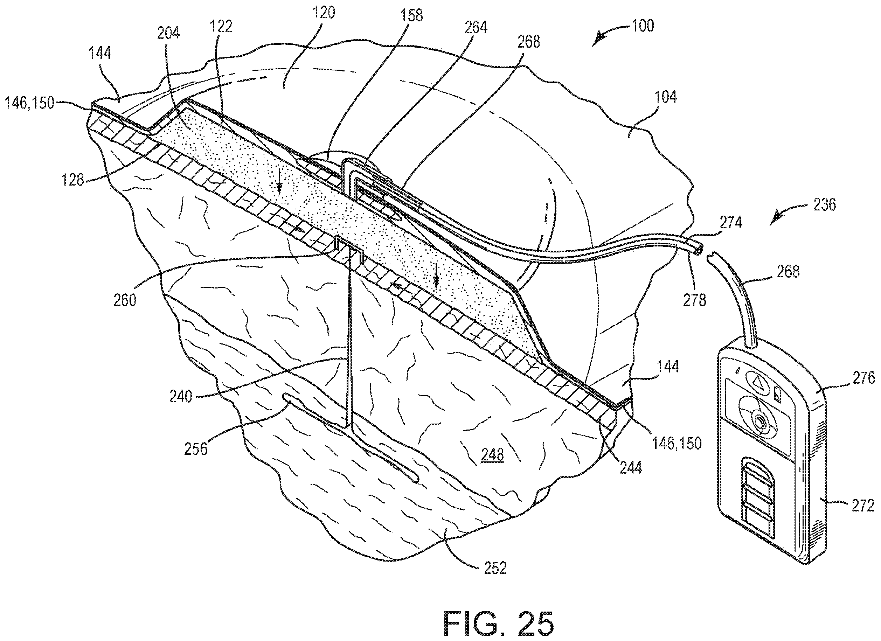

Referring now to FIGS. 22-25 dressing 36, is shown, according to an exemplary embodiment. FIG. 22 is a front view of dressing 36. FIG. 23 is a perspective view of dressing 36. FIG. 24 is an exploded view illustrating several layers 120 - 154 of dressing 36. FIG. 25 is a cross-sectional view of dressing 36 adhered to a surface 104, such as a patient's torso, knee, or elbow.

In various embodiments, dressing 36 can be formed as a substantially flat sheet for topical application to wounds. Dressing 36 can lie flat for treatment of substantially flat wounds and is also configured to bend to conform to body surfaces having high curvature, such as breasts, or body surfaces at joints (e.g., at elbows and knees as shown in FIG. 1). Dressing 36 has a profile or a perimeter that is generally heart-shaped and includes a first lobe 108 (e.g. convex portion) and a second lobe 112 (e.g. convex portion) that define a concave portion 116 therebetween. Dressing 36 is generally symmetric about an axis A. It is contemplated that the size of the wound dressing can range from 180 cm$^2$ to 1000 cm$^2$. More preferably, the size of the wound dressing can range from 370 cm$^2$ to 380 cm$^2$, 485 cm$^2$ to 495 cm$^2$, and/or 720 cm$^2$ to 740 cm$^2$. However, other shapes and sizes of wound dressing 36 are also possible depending on the intended use. For example, for some uses, dressing 36 may have asymmetrically-shaped lobes 108, 112.

Dressing 36 is shown to include a plurality of layers, including a drape layer 120 (e.g., drape 18), a manifold layer 124, a wound-interface layer 128, a rigid support layer 142, a first adhesive layer 146, a second adhesive layer 150, and a patient-contacting layer 154. In some embodiments, dressing 36 includes a removable cover sheet 132 to cover the manifold layer 124, the wound-interface layer 128, the second adhesive layer 150, and/or the patient-contacting layer 154 before use.

## Drape Layer

The drape layer 120 is shown to include a first surface 136 and a second, wound-facing, surface 140 opposite the first surface 136. When dressing 36 is applied to a wound, the first surface 136 faces away from the wound, whereas the second surface 140 faces toward the wound. The drape layer 120 supports the manifold layer 124 and the wound-interface layer 128 and provides a barrier to passage of microorganisms through dressing 36. The drape layer 120 is configured to provide a sealed space over a wound or incision. In some embodiments, the drape layer 120 is an elastomeric material or may be any material that provides a fluid seal. "Fluid seal" means a seal adequate to hold pressure at a desired site given the particular reduced-pressure subsystem involved. The term "elastomeric" means having the properties of an elastomer and generally refers to a polymeric material that has rubber-like properties. Examples of elastomers may include, but are not limited to, natural rubbers, polyisoprene, styrene butadiene rubber, chloroprene rubber, polybutadiene, nitrile rubber, butyl rubber, ethylene propylene rubber, ethylene propylene diene monomer, chlorosulfonated polyethylene, polysulfide rubber, polyurethane, EVA film, co-polyester, and silicones. As nonlimiting examples, the drape layer 120 may be formed from materials that include a silicone, 3M Tegaderm® drape material, acrylic drape material such as one available from Avery, or an incise drape material.

The drape layer 120 may be substantially impermeable to liquid and substantially permeable to water vapor. In other words, the drape layer 120 may be permeable to water vapor, but not permeable to liquid water or wound exudate. This increases the total fluid handling capacity (TFHC) of wound dressing 36 while promoting a moist wound environment. In some embodiments, the drape layer 120 is also impermeable to bacteria and other microorganisms. In some embodiments, the drape layer 120 is configured to wick moisture from the manifold layer 124 and distribute the moisture across the first surface 136.

In the illustrated embodiment, the drape layer 120 defines a cavity 122 (FIG. 25) for receiving the manifold layer 124, the wound-interface layer 128, and the first adhesive layer 146. As shown in FIG. 23, the manifold layer 124, the wound-interface layer 128, and the first adhesive layer 146 can have a similar perimeter or profile. In some embodiments, a perimeter of the drape layer 120 extends beyond (e.g. circumscribes) the perimeter of the manifold layer 124 to provide a margin 144. The first adhesive layer 146 includes a first surface 147 and a second, wound-facing surface 149. Both first surface 147 and the second surface 149 are coated with an adhesive, such as an acrylic adhesive, a silicone adhesive, and/or other adhesives. The first surface 147 of the first adhesive layer 146 is secured to the second surface 224 of the wound-interface layer 128. The second surface 149 of the first adhesive layer 146 is secured to the second adhesive layer 150. The second adhesive layer 150 includes a first surface 151 and a second, wound-facing surface 153. The second surface 149 of the first adhesive layer 146 is secured to the first surface 151 of the second adhesive layer 150. The second surface 153 of the second adhesive layer 150 is coated with an acrylic adhesive, a silicone adhesive, and/or other adhesives. The adhesive applied to the second surface 153 of the second adhesive layer 150 is intended to ensure that dressing 36 adheres to the surface 104 of the patient's skin (as shown in FIG. 25) and that dressing 36 remains in place throughout the wear time. The second adhesive layer 150 has a perimeter or profile that is similar to a perimeter or profile of the margin 144. In the illustrated embodiment, the first surface 151 of the second adhesive layer 150 is welded to the margin 144. In other embodiments, the first surface 151 of the second adhesive layer is secured to the margin 144 using an adhesive, such as an acrylic adhesive, a silicone adhesive, or another type of adhesive. The patient-contacting layer 154 includes a first surface 155 and a second, wound-facing surface 157. In some embodiments, the patient-contacting layer 154 can be made of a hydrocolloid material, a silicone material or another similar material. The first surface 155 of the patient-contacting layer 154 can be secured to the second adhesive layer 150. The patient-contacting layer 154 follows a perimeter of the manifold layer 124. In some embodiments, the patient-contacting layer 154 can be made of a polyurethane film coated with an acrylic or silicone adhesive on both surfaces 155, 157. In some embodiments, the patient-contacting layer 154 can include a hydrocolloid adhesive on the second, wound-facing, surface 157. The margin 144 and/or the second adhesive layer 150 may extend around all sides of the manifold layer 124 such that dressing 36 is a so-called island dressing. In other embodiments, the margin 144 and/or the second adhesive layer 150 can be eliminated and dressing 36 can be adhered to the surface 104 using other techniques. In some embodiments, the first adhesive layer 146, the second adhesive layer 150, and the patient-contacting layer 154 can collectively form a base layer that includes an adhesive on both sides that is (i) configured to secure the drape layer 120 to the manifold layer 124, the optional wound-interface layer 128, and (ii) configured to secure dressing 36 to a patient's tissue. In some embodiments, the base layer can be integrally formed with the drape layer 120. In some embodiments, the base layer can be a layer of a polyurethane film having a first surface and a second, wound-facing surface. Both the first surface and the second surface can be coated with an adhesive (such as an acrylic or silicone adhesive). In some embodiments, the wound-facing surface of the base layer can include a hydrocolloid adhesive.

In some embodiments, a reduced-pressure interface 158 can be integrated with the drape layer 120. The reduced-pressure interface 158 can be in fluid communication with the negative pressure system through a removed fluid conduit 268 (FIG. 25). The reduced-pressure interface 158 is configured to allow fluid communication between a negative pressure source and dressing 36 (e.g., through the drape layer 120) via a removed fluid conduit coupled between the reduced-pressure interface 158 and the negative pressure source such that negative pressure generated by the negative pressure source can be applied to dressing 36 (e.g., through the drape layer 120). In some embodiments, the reduced-pressure interface 158 can be integrated (e.g., integrally formed) with the drape layer 120. In other embodiments, the reduced-pressure interface 158 can be separate from the drape layer 120 and configured to be coupled to the drape layer 120 by a user.

With continued reference to FIG. 23, the rigid support layer 142 is positioned above the first surface 136 of the drape layer 120. The rigid support layer 142 is spaced from but proximate the margin 144 and the second adhesive layer 150. The rigid support layer 142 is made of a rigid material and helps dressing 36 maintain rigidity before dressing 36 is secured to the surface 104 of the patient. The rigid support layer 142 is intended to be removed from the drape layer 120 after dressing 36 has been secured to the surface 104 of the patient.

In some embodiments, the second surface 140 of the drape layer 120 contacts the manifold layer 124. The second surface 140 of the drape layer 120 may be adhered to the manifold layer 124 or may simply contact the manifold layer 124 without the use of an adhesive.

In some embodiments, the adhesive applied to the second surface 140 of the drape layer 120 is moisture vapor transmitting and/or patterned to allow passage of water vapor therethrough. The adhesive may include a continuous moisture vapor transmitting, pressure-sensitive adhesive layer of the type conventionally used for island-type wound dressings (e.g. a polyurethane-based pressure sensitive adhesive).

Manifold layer

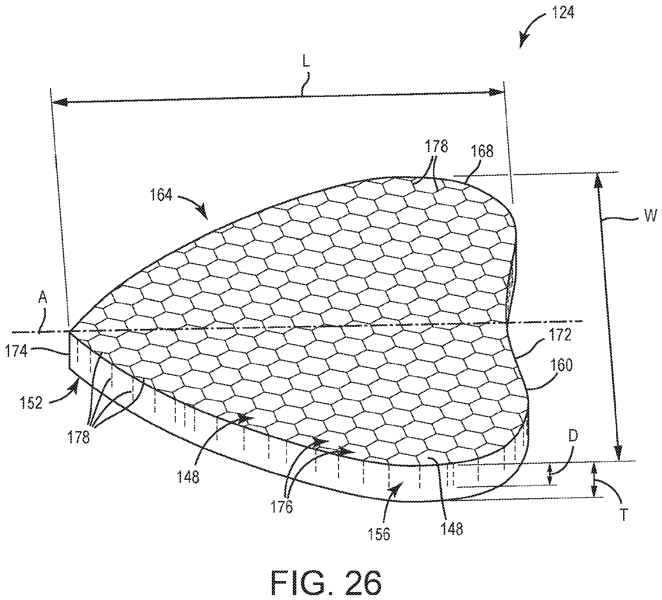

Referring to FIG. 26, the manifold layer 124 is shown to include a first surface 148 and a second, wound-facing surface 152 opposite the first surface 148. When dressing 36 is applied to a wound, the first surface 148 faces away from the wound, whereas the second surface 152 faces toward the wound. In some embodiments, the first surface 148 of the manifold layer 124 contacts the second surface 140 of the drape layer 120. In some embodiments, the second surface 152 of the manifold layer 124 contacts the wound-interface layer 128. The manifold layer 124 is configured for transmission of negative pressure to the patient's tissue at and/or proximate a wound and/or incision. The manifold layer 124 is configured to wick fluid (e.g. exudate) from the wound and includes in-molded manifold layer structures for distributing negative pressure throughout dressing 36 during negative pressure wound therapy treatments.

The manifold layer 124 can be made from a porous and permeable foam-like material and, more particularly, a reticulated, open-cell polyurethane or polyether foam that allows good permeability of wound fluids while under a reduced pressure. One such foam material that has been used is the V.A.C.® Granufoam™ material that is available from Kinetic Concepts, Inc. (KCI) of San Antonio, Tex. Any material or combination of materials might be used for the manifold layer 124 provided that the manifold layer 124 is operable to distribute the reduced pressure and provide a distributed compressive force along the wound site.

The reticulated pores of the Granufoam™ material that are in the range from about 400 to 600 microns, are preferred, but other materials may be used. The density of the manifold layer material, e.g., Granufoam™ material, is typically in the range of about 1.3 lb/ft$^3$-1.6 lb/ft$^3$ (20.8 kg/m$^3$-25.6 kg/m$^3$). A material with a higher density (smaller pore size) than Granufoam™ material may be desirable in some situations. For example, the Granufoam™ material or similar material with a density greater than 1.6 lb/ft$^3$ (25.6 kg/m$^3$) may be used. As another example, the Granufoam™ material or similar material with a density greater than 2.0 lb/ft$^3$ (32 kg/m$^3$) or 5.0 lb/ft$^3$ (80.1 kg/m$^3$) or even more may be used. The more dense the material is, the higher compressive force that may be generated for a given reduced pressure. If a foam with a density less than the tissue at the tissue site is used as the manifold layer material, a lifting force may be developed. In one illustrative embodiment, a portion, e.g., the edges, of dressing 36 may exert a compressive force while another portion, e.g., a central portion, may provide a lifting force.

The manifold layer material may be a reticulated foam that is later felted to thickness of about one third (⅓) of the foam's original thickness. Among the many possible manifold layer materials, the following may be used: Granufoam™ material or a Foamex® technical foam (www.foamex.com). In some instances it may be desirable to add ionic silver to the foam in a microbonding process or to add other substances to the manifold layer material such as antimicrobial agents. The manifold layer material may be isotropic or anisotropic depending on the exact orientation of the compressive forces that are desired during the application of reduced pressure. The manifold layer material may also be a bio-absorbable material.

As shown in FIGS. 22-24 and 26, the manifold layer 124 is generally symmetrical, heart-shaped, and includes a first convex curved side 156 defining a first lobe 160, a second convex curved side 164 defining a second lobe 168, and a concave connecting portion 172 extending therebetween. The manifold layer 124 can have a width W ranging between 8 cm and 33 cm, and more preferably between 17 cm and 33 cm. The manifold layer 124 can have a length L ranging between 7 cm and 35 cm, and more preferably between 14 cm and 30 cm. The manifold layer 124 can have a thickness T ranging between 14 mm and 24 mm, and more preferably 19 mm. The first lobe 160 and the second lobe 168 are convex and can have a radius of curvature ranging between 3 cm and 10 cm, and more preferably from 5 cm to 9 cm. The connecting portion 172 is generally concave and can have a radius of curvature ranging between 20 cm and 33 cm, and more preferably from 22 cm to 28 cm. The first curved side 156 and the second curved side 164 form a point 174 positioned generally opposite the connecting portion 172. In the illustrated embodiment, the first curved side 156 and the second curved side 164 are generally symmetric about the axis A.

**Wound Therapy System**

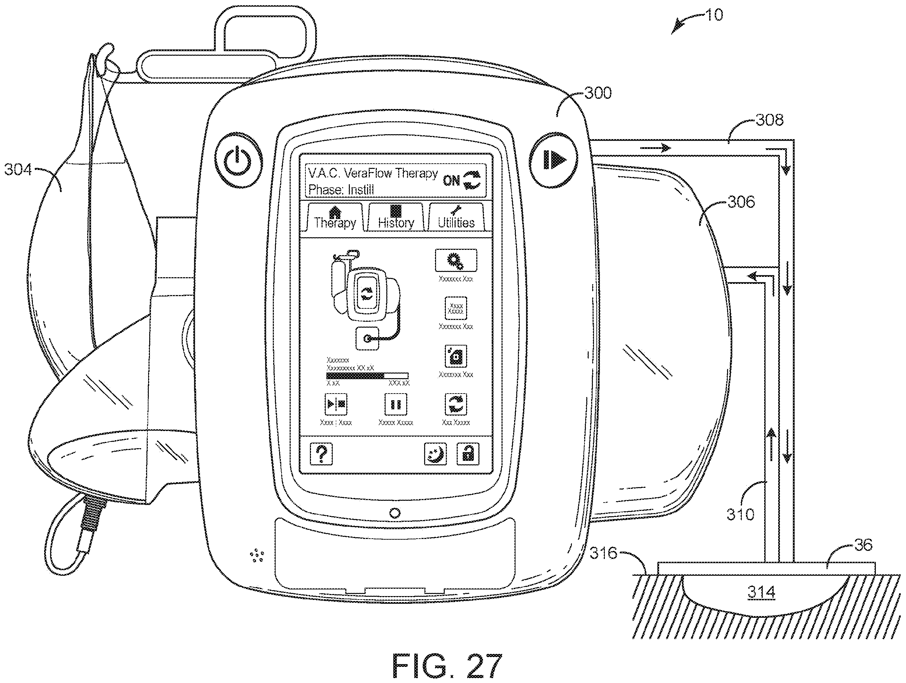

Referring now to FIGS. 27-31, system 10 is shown, according to an exemplary embodiment. System 10 is shown to include a therapy device 300 fluidly connected to a dressing 36 via tubing 308 and 110. Dressing 36 may be adhered or sealed to a patient's skin 316 surrounding a wound 314. Several examples of wound dressings 36 which can be used in combination with system 10 are described in detail in U.S. Patent No. 7,651,484 granted January 26, 2010, U.S. Patent No. 8,394,081 granted March 12, 2013, and U.S. Patent Application No. 14/087,418 filed November 22, 2013.

Therapy device 300 can be configured to provide negative pressure wound therapy by reducing the pressure at wound 314. Therapy device 300 can draw a vacuum at wound 314 (relative to atmospheric pressure) by removing wound exudate, air, and other fluids from wound 314. Wound exudate may include fluid that filters from a patient's circulatory system into lesions or areas of inflammation. For example, wound exudate may include water and dissolved solutes such as blood, plasma proteins, white blood cells, platelets, and red blood cells. Other fluids removed from wound 314 may include instillation fluid 305 previously delivered to wound 314. Instillation fluid 305 can include, for example, a cleansing fluid, a prescribed fluid, a medicated fluid, an antibiotic fluid, or any other type of fluid which can be delivered to wound 314 during wound treatment. Instillation fluid 305 may be held in an instillation fluid canister 304 and controllably dispensed to wound 314 via instillation fluid tubing 308. In some embodiments, instillation fluid canister 304 is detachable from therapy device 300 to allow canister 306 to be refilled and replaced as needed.

The fluids 307 removed from wound 314 pass through removed fluid tubing 310 and are collected in removed fluid canister 306. Removed fluid canister 306 may be a component of therapy device 300 configured to collect wound exudate and other fluids 307 removed from wound 314. In some embodiments, removed fluid canister 306 is detachable from therapy device 300 to allow canister 306 to be emptied and replaced as needed. A lower portion of canister 306 may be filled with wound exudate and other fluids 307 removed from wound 314, whereas an upper portion of canister 306 may be filled with air. Therapy device 300 can be configured to draw a vacuum within canister 306 by pumping air out of canister 306. The reduced pressure within canister 306 can be translated to dressing 36 and wound 314 via tubing 310 such that dressing 36 and wound 314 are maintained at the same pressure as canister 306.

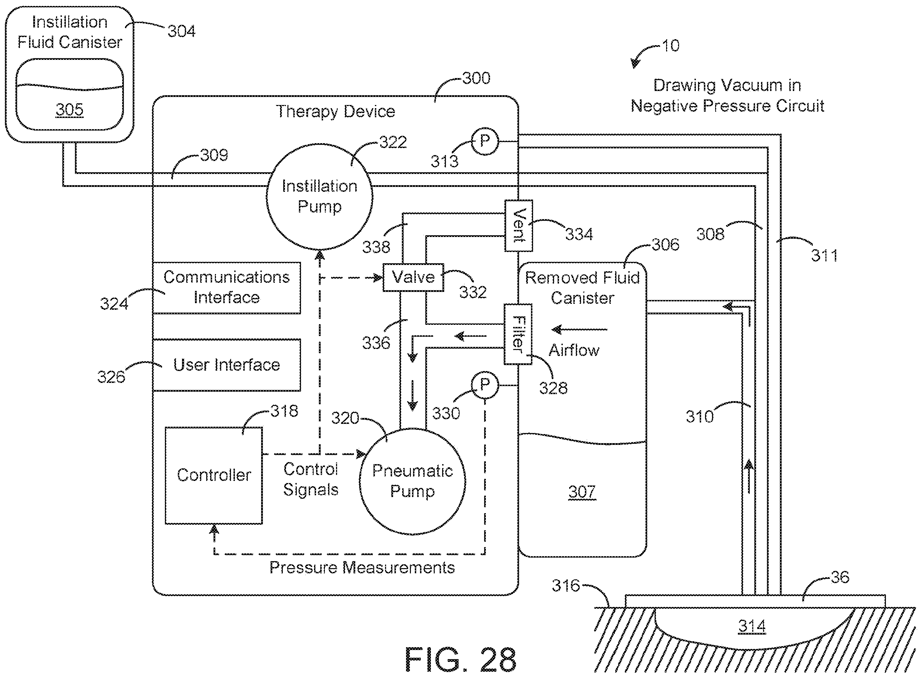

Referring particularly to FIGS. 28-29, block diagrams illustrating therapy device 300 in greater detail are shown, according to an exemplary embodiment. Therapy device 300 is shown to include a pneumatic pump 320, an instillation pump 322, a valve 332, a filter 328, and a controller 318. Pneumatic pump 320 can be fluidly coupled to removed fluid canister 306 (e.g., via conduit 336) and can be configured to draw a vacuum within canister 306 by pumping air out of canister 306. In some embodiments, pneumatic pump 320 is configured to operate in both a forward direction and a reverse direction. For example, pneumatic pump 320 can operate in the forward direction to pump air out of canister 306 and decrease the pressure within canister 306. Pneumatic pump 320 can operate in the reverse direction to pump air into canister 306 and increase the pressure within canister 306. Pneumatic pump 320 can be controlled by controller 318, described in greater detail below.

Similarly, instillation pump 322 can be fluidly coupled to instillation fluid canister 304 via tubing 309 and fluidly coupled to dressing 36 via tubing 308. Instillation pump 322 can be operated to deliver instillation fluid 305 to dressing 36 and wound 314 by pumping instillation fluid 305 through tubing 309 and tubing 308, as shown in FIG. 31. Instillation pump 322 can be controlled by controller 318, described in greater detail below.

Filter 328 can be positioned between removed fluid canister 306 and pneumatic pump 320 (e.g., along conduit 336) such that the air pumped out of canister 306 passes through filter 328. Filter 328 can be configured to prevent liquid or solid particles from entering conduit 336 and reaching pneumatic pump 320. Filter 328 may include, for example, a bacterial filter that is hydrophobic and/or lipophilic such that aqueous and/or oily liquids will bead on the surface of filter 328. Pneumatic pump 320 can be configured to provide sufficient airflow through filter 328 that the pressure drop across filter 328 is not substantial (e.g., such that the pressure drop will not substantially interfere with the application of negative pressure to wound 314 from therapy device 300).

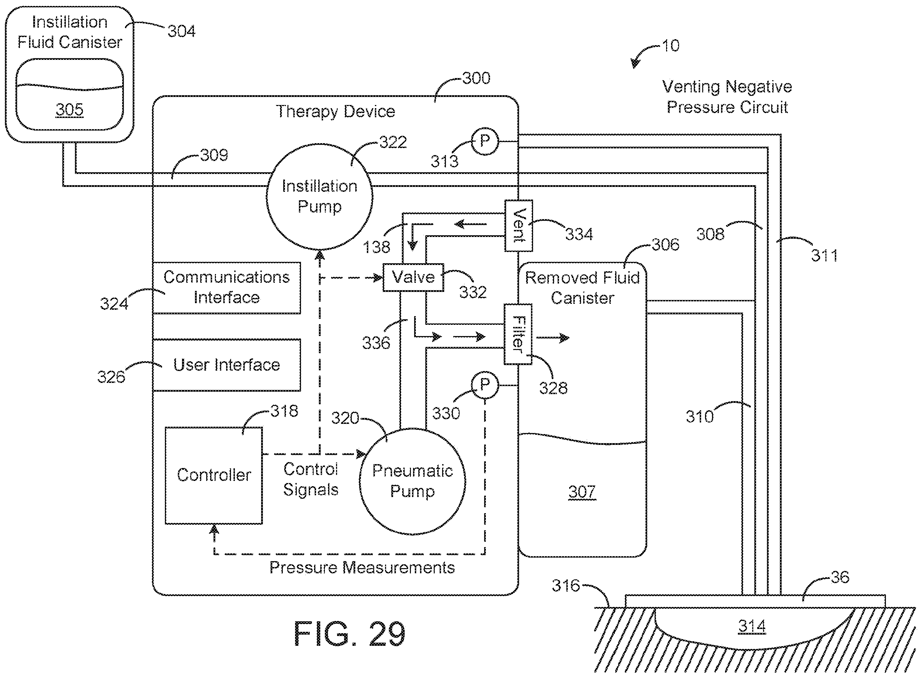

In some embodiments, therapy device 300 operates a valve 332 to controllably vent the negative pressure circuit, as shown in FIG. 29. Valve 332 can be fluidly connected with pneumatic pump 320 and filter 328 via conduit 336. In some embodiments, valve 332 is configured to control airflow between conduit 336 and the environment around therapy device 300. For example, valve 332 can be opened to allow airflow into conduit 336 via vent 334 and conduit 338, and closed to prevent airflow into conduit 336 via vent 334 and conduit 338. Valve 332 can be opened and closed by controller 318, described in greater detail below. When valve 332 is closed, pneumatic pump 320 can draw a vacuum within a negative pressure circuit by causing airflow through filter 328 in a first direction, as shown in FIG. 28. The negative pressure circuit may include any component of system 10 that can be maintained at a negative pressure when performing negative pressure wound therapy (e.g., conduit 336, removed fluid canister 306, tubing 310, dressing 36, and/or wound 314). For example, the negative pressure circuit may include conduit 336, removed fluid canister 306, tubing 310, dressing 36, and/or wound 314. When valve 332 is open, airflow from the environment around therapy device 300 may enter conduit 336 via vent 334 and conduit 338 and fill the vacuum within the negative pressure circuit. The airflow from conduit 336 into canister 306 and other volumes within the negative pressure circuit may pass through filter 328 in a second direction, opposite the first direction, as shown in FIG. 29.

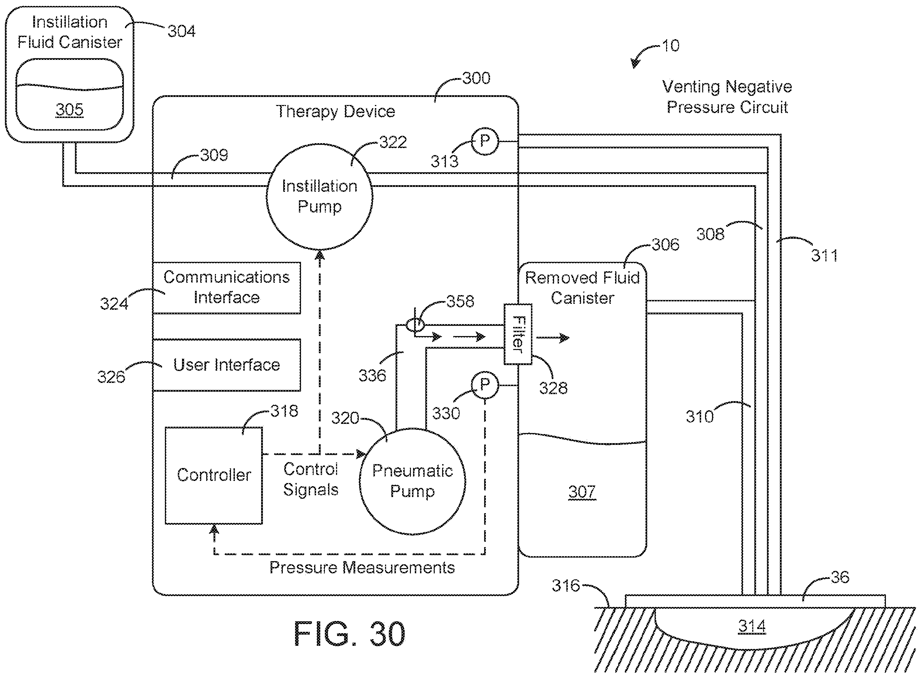

In some embodiments, therapy device 300 vents the negative pressure circuit via an orifice 358, as shown in FIG. 30. Orifice 358 may be a small opening in conduit 336 or any other component of the negative pressure circuit (e.g., removed fluid canister 306, tubing 310, tubing 311, dressing 36, etc.) and may allow air to leak into the negative pressure circuit at a known rate. In some embodiments, therapy device 300 vents the negative pressure circuit via orifice 358 rather than operating valve 332. Valve 332 can be omitted from therapy device 300 for any embodiment in which orifice 358 is included. The rate at which air leaks into the negative pressure circuit via orifice 358 may be substantially constant or may vary as a function of the negative pressure, depending on the geometry of orifice 358.

In some embodiments, therapy device 300 includes a variety of sensors. For example, therapy device 300 is shown to include a pressure sensor 330 configured to measure the pressure within canister 306 and/or the pressure at dressing 36 or wound 314. In some embodiments, therapy device 300 includes a pressure sensor 313 configured to measure the pressure within tubing 311. Tubing 311 may be connected to dressing 36 and may be dedicated to measuring the pressure at dressing 36 or wound 314 without having a secondary function such as channeling installation fluid 305 or wound exudate. In various embodiments, tubing 308, 110, and 111 may be physically separate tubes or separate lumens within a single tube that connects therapy device 300 to dressing 36. Accordingly, tubing 310 may be described as a negative pressure lumen that functions apply negative pressure dressing 36 or wound 314, whereas tubing 311 may be described as a sensing lumen configured to sense the pressure at dressing 36 or wound 314. Pressure sensors 330 and 313 can be located within therapy device 300, positioned at any location along tubing 308, 110, and 111, or located at dressing 36 in various embodiments. Pressure measurements recorded by pressure sensors 330 and/or 313 can be communicated to controller 318. Controller 318 use the pressure measurements as inputs to various pressure testing operations and control operations performed by controller 318.

Controller 318 can be configured to operate pneumatic pump 320, instillation pump 322, valve 332, and/or other controllable components of therapy device 300. For example, controller 318 may instruct valve 332 to close and operate pneumatic pump 320 to establish negative pressure within the negative pressure circuit. Once the negative pressure has been established, controller 318 may deactivate pneumatic pump 320. Controller 318 may cause valve 332 to open for a predetermined amount of time and then close after the predetermined amount of time has elapsed.

In some embodiments, therapy device 300 includes a user interface 326. User interface 326 may include one or more buttons, dials, sliders, keys, or other input devices configured to receive input from a user. User interface 326 may also include one or more display devices (e.g., LEDs, LCD displays, etc.), speakers, tactile feedback devices, or other output devices configured to provide information to a user. In some embodiments, the pressure measurements recorded by pressure sensors 330 and/or 313 are presented to a user via user interface 326. User interface 326 can also display alerts generated by controller 318. For example, controller 318 can generate a "no canister" alert if canister 306 is not detected.

In some embodiments, therapy device 300 includes a data communications interface 324 (e.g., a USB port, a wireless transceiver, etc.) configured to receive and transmit data. Communications interface 324 may include wired or wireless communications interfaces (e.g., jacks, antennas, transmitters, receivers, transceivers, wire terminals, etc.) for conducting data communications external systems or devices. In various embodiments, the communications may be direct (e.g., local wired or wireless communications) or via a communications network (e.g., a WAN, the Internet, a cellular network, etc.). For example, communications interface 324 can include a USB port or an Ethernet card and port for sending and receiving data via an Ethernet-based communications link or network. In another example, communications interface 324 can include a Wi-Fi transceiver for communicating via a wireless communications network or cellular or mobile phone communications transceivers.

**Configuration of Exemplary Embodiments**

The construction and arrangement of the systems and methods as shown in the various exemplary embodiments are illustrative only. Although only a few embodiments have been described in detail in this disclosure, many modifications are possible (e.g., variations in sizes, dimensions, structures, shapes and proportions of the various elements, values of parameters, mounting arrangements, use of materials, colors, orientations, etc.). For example, the position of elements can be reversed or otherwise varied and the nature or number of discrete elements or positions can be altered or varied. Accordingly, all such modifications are intended to be included within the scope of the present disclosure. The order or sequence of any process or method steps can be varied or resequenced according to alternative embodiments. Other substitutions, modifications, changes, and omissions can be made in the design, operating conditions and arrangement of the exemplary embodiments without departing from the scope of the present disclosure, the invention being defined by the appended claims.

The present disclosure contemplates methods, systems and program products on any machine-readable media for accomplishing various operations. The embodiments of the present disclosure can be implemented using existing computer processors, or by a special purpose computer processor for an appropriate system, incorporated for this or another purpose, or by a hardwired system. Embodiments within the scope of the present disclosure include program products comprising machine-readable media for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, or any other medium which can be used to carry or store desired program code in the form of machine-executable instructions or data structures and which can be accessed by a general purpose or special purpose computer or other machine with a processor. Combinations of the above are also included within the scope of machine-readable media. Machine-executable instructions include, for example, instructions and data which cause a general purpose computer, special purpose computer, or special purpose processing machines to perform a certain function or group of functions.

Although the figures show a specific order of method steps, the order of the steps may differ from what is depicted. Also two or more steps can be performed concurrently or with partial concurrence. Such variation will depend on the software and hardware systems chosen and on designer choice. All such variations are within the scope of the disclosure. Likewise, software implementations could be accomplished with standard programming techniques with rule based logic and other logic to accomplish the various connection steps, processing steps, comparison steps and decision steps.

**Claims**

1. A system (10) for calculating range of motion of a patient's jointed limb, the system (10) comprising:

   a drape (18) suitable for being adhered to a patient's skin (32) of the jointed limb, wherein the drape (18) comprises a plurality of locators (20), wherein, in use, one or more of the locators (20a) are positioned at an upper limb (14) of the jointed limb, one or more of the locators (20c) are positioned at a lower limb (16) of the jointed limb, one or more of the locators (20b) are positioned at a joint (12) of the jointed limb between the upper limb (14) and the lower limb (16), a first centerline (26) extends between a center of a first of the one or more locators (20c) positioned at the lower limb (16) and a center of a first of the one or more locators (20b) positioned at the joint (12), and a second centerline (24) extends between a center of a first of the one or more locators (20a) positioned at the upper limb (14) and the center of the first of the one or more locators (20b) positioned at the joint (12);
   a personal computer device (100) comprising an imaging device (614), wherein the personal computer device (100) is configured to:

      record (1402) a first image of the patient's joint in a fully extended position with the imaging device (614);
      record (1402) a second image of the patient's joint in a fully flexed position with the imaging device (614);

identify (1404) positions of the locators (20) of both the first image and the second image;

determine (1410) an extended angle of the patient's joint based on the identified positions of the locators of the first image, wherein the extended angle is an angle between the first centerline and the second centerline when the patient's joint in the fully extended position;

determine (1408) a flexed angle of the patient's joint based on the identified positions of the locators of the second image, wherein the flexed angle is an angle between the first centerline and the second centerline when the patient's joint in the fully flexed position; and

determine (1412) a range of motion angle based on the extended angle and the flexed angle; and

perform a calibration process to determine offset amounts for any of the flexed angle, the extended angle, and the range of motion angle to account for orientation of the imaging device relative to the jointed limb, the calibration process comprising:

analyzing the first image and the second image to determine a difference in a shape of the locators (20) relative to a known shape of the locators (20);
determining an orientation of the imaging device (614) relative to the jointed limb based on the difference in the shape of the locators (20); and
determining an offset amount for any of the flexed angle, the extended angle, and the range of motion angle to account for the orientation of the imaging device (614) relative to the jointed limb.

2. The system of Claim 1, wherein the personal computer device (100) is a mobile device with an application configured to determine the range of motion angle.

3. The system of Claim 1, wherein the positions of the locators (20) of both the first image and the second image are identified based on image data of the first image and the second image.

4. The system of Claim 1, wherein the personal computer device (100) is configured to generate a report and control a display screen to display the report.

5. The system of Claim 4, wherein the report comprises any of the range of motion angle, tabular historical information of the range of motion angle, graphical historical information of the range of motion angle, and improvements in the range of motion angle over time.

6. The system of Claim 4, wherein the personal computer device (100) is configured to provide the report to a clinician device (612).

7. The system of Claim 1, wherein the difference in the shape of the locators (20) is determined based on one or more initially recorded images.

8. The system of Claim 1, wherein the personal computer device (100) is configured to provide a notification to the patient to record the first image and the second image.

9. The system of Claim 1, wherein the system (10) comprises a negative pressure source (320) configured to draw a negative pressure at a wound (314) at the jointed limb.

10. The system of Claim 9, wherein the system (10) further comprises a canister (306) configured to receive wound exudate.

11. The system of Claim 10, wherein the system (10) further comprises a connector (34) at the drape (18) and a tubular member (30), wherein the connector (34) is configured to fluidly couple the wound (314) with the negative pressure source (320) through the tubular member (30, 310).

12. The system of Claim 1, wherein the system (10) comprises a dressing (36), the dressing (36) comprising the drape (18) and a manifold layer (124).

**Patentansprüche**

1. Ein System (10) zum Berechnen eines Bewegungsbereichs eines Glieds mit Gelenk eines Patienten, das System (10), aufweisend:

ein Abdecktuch (18), das zum Aufkleben auf die Haut (32) des Glieds mit Gelenk eines Patienten geeignet ist, wobei das Abdecktuch (18) eine Mehrzahl von Lokatoren (20) aufweist, wobei, in Verwendung, einer oder mehrere der Lokatoren (20a) an einem oberen Glied (14) des Glieds mit Gelenk positioniert sind, einer oder mehrere der Lokatoren (20c) an einem unteren Glied (16) des Glieds mit Gelenk positioniert sind, einer oder mehrere der Lokatoren (20b) an einem Gelenk (12) des Glieds mit Gelenk zwischen dem oberen Glied (14) und dem unteren Glied (16) positioniert sind, sich eine erste Mittellinie (26) zwischen einer Mitte eines ersten des einen oder der mehreren Lokatoren (20c), die an dem unteren Glied (16) positioniert sind, und einer Mitte eines ersten des einen oder der mehreren Lokatoren (20b), die an dem Gelenk (12) positioniert sind, erstreckt, und sich eine zweite Mittellinie (24) zwischen einer Mitte eines ersten des einen oder der mehreren Lokatoren (20a), die an dem oberen Glied (14) positioniert sind, und der Mitte des ersten des einen oder der mehreren Lokatoren, die (20b) an dem Gelenk (12) positioniert sind, erstreckt;

eine Personalcomputervorrichtung (100), aufweisend eine Bildgebungsvorrichtung (614), wobei die Personalcomputervorrichtung (100) konfiguriert ist zum:

> Aufzeichnen (1402) eines ersten Bildes des Gelenks des Patienten in einer vollständig gestreckten Position mit der Bildgebungsvorrichtung (614);
> Aufzeichnen (1402) eines zweiten Bildes des Gelenks des Patienten in einer vollständig gebeugten Position mit der Bildgebungsvorrichtung (614);
> Identifizieren (1404) von Positionen der Lokatoren (20) sowohl des ersten Bildes als auch des zweiten Bildes;
> Bestimmen (1410) eines gestreckten Winkels des Gelenks des Patienten basierend auf den identifizierten Positionen der Lokatoren des ersten Bildes, wobei der gestreckte Winkel ein Winkel zwischen der ersten Mittellinie und der zweiten Mittellinie ist, wenn das Gelenk des Patienten in der vollständig gestreckten Position ist;
> Bestimmen (1408) eines Beugewinkels des Gelenks des Patienten basierend auf den identifizierten Positionen der Lokatoren des zweiten Bildes, wobei der Beugewinkel ein Winkel zwischen der ersten Mittellinie und der zweiten Mittellinie ist, wenn das Gelenk des Patienten in der vollständig gebeugten Position ist; und
> Bestimmen (1412) eines Bewegungsbereichswinkels basierend auf dem gestreckten Winkel und dem Beugewinkel; und
> Durchführen eines Kalibrierungsverfahrens, um Versatzbeträge für einen beliebigen des Beugewinkels, des gestreckten Winkels und des Bewegungsbereichswinkels zu bestimmen, um eine Ausrichtung der Bildgebungsvorrichtung relativ zu dem Glied mit Gelenk zu berücksichtigen, das Kalibrierungsverfahren aufweisend:

>> Analysieren des ersten Bildes und des zweiten Bildes, um einen Unterschied in einer Form der Lokatoren (20) relativ zu einer bekannten Form der Lokatoren (20) zu bestimmen;
Bestimmen einer Ausrichtung der Bildgebungsvorrichtung (614) relativ zu dem Glied mit Gelenk basierend auf dem Unterschied in der Form der Lokatoren (20); und
Bestimmen eines Versatzbetrags für einen beliebigen des Beugewinkels, des gestreckten Winkels und des Bewegungsbereichswinkels, um die Ausrichtung der Bildgebungsvorrichtung (614) relativ zu dem Glied mit Gelenk zu berücksichtigen.

2. Das System nach Anspruch 1, wobei die Personalcomputervorrichtung (100) eine mobile Vorrichtung mit einer Anwendung ist, die konfiguriert ist, um den Bewegungsbereichswinkel zu bestimmen.

3. Das System nach Anspruch 1, wobei die Positionen der Lokatoren (20) sowohl des ersten Bildes als auch des zweiten Bildes basierend auf Bilddaten des ersten Bildes und des zweiten Bildes identifiziert werden.

4. Das System nach Anspruch 1, wobei die Personalcomputervorrichtung (100) konfiguriert ist, um einen Bericht zu erzeugen und einen Anzeigebildschirm zu steuern, um den Bericht anzuzeigen.

5. Das System nach Anspruch 4, wobei der Bericht ein beliebiges von dem Bewegungsbereichswinkel, tabellarischen Verlaufsinformationen des Bewegungsbereichswinkels, grafischen Verlaufsinformationen des Bewegungsbereichswinkels und Verbesserungen des Bewegungsbereichswinkels im Laufe der Zeit aufweist.

6. Das System nach Anspruch 4, wobei die Personalcomputervorrichtung (100) konfiguriert ist, um den Bericht einer Klinikarztvorrichtung (612) bereitzustellen.

7. Das System nach Anspruch 1, wobei der Unterschied in der Form der Lokatoren (20) basierend auf einem oder mehreren anfänglich aufgezeichneten Bildern bestimmt wird.

8. Das System nach Anspruch 1, wobei die Personalcomputervorrichtung (100) konfiguriert ist, um dem Patienten eine Benachrichtigung bereitzustellen, das erste Bild und das zweite Bild aufzuzeichnen.

9. Das System nach Anspruch 1, wobei das System (10) eine Unterdruckquelle (320), die konfiguriert ist, um einen Unterdruck an einer Wunde (314) an dem Glied mit Gelenk auszuüben, aufweist.

**10.** Das System nach Anspruch 9, wobei das System (10) ferner einen Behälter (306), der konfiguriert ist, um Wundexsudat aufzunehmen, aufweist.

**11.** Das System nach Anspruch 10, wobei das System (10) ferner einen Verbinder (34) an dem Abdecktuch (18) und ein röhrenförmiges Element (30) aufweist, wobei der Verbinder (34) konfiguriert ist, um die Wunde (314) mit der Unterdruckquelle (320) durch das röhrenförmige Element (30, 310) fluidisch zu koppeln.

**12.** Das System nach Anspruch 1, wobei das System (10) einen Verband (36) aufweist, der Verband (36) aufweisend das Abdecktuch (18) und eine Verteilerschicht (124).

**Revendications**

**1.** Système (10) permettant de calculer une amplitude de mouvement d'un membre articulé d'un patient, le système (10) comprenant :

un champ (18) adapté à être collé à la peau d'un patient (32) du membre articulé, dans lequel le champ (18) comprend une pluralité de localisateurs (20), dans lequel, en cours d'utilisation, un ou plusieurs des localisateurs (20a) sont positionnés au niveau d'un membre supérieur (14) du membre articulé, un ou plusieurs des localisateurs (20c) sont positionnés au niveau d'un membre inférieur (16) du membre articulé, un ou plusieurs des localisateurs (20b) sont positionnés au niveau d'une articulation (12) du membre articulé entre le membre supérieur (14) et le membre inférieur (16), une première ligne médiane (26) s'étend entre un centre d'un premier du ou des localisateurs (20c) positionnés au niveau du membre inférieur (16) et un centre d'un premier du ou des localisateurs (20b) positionnés au niveau de l'articulation (12), et une seconde ligne médiane (24) s'étend entre un centre d'un premier du ou des localisateurs (20a) positionnés au niveau du membre supérieur (14) et le centre du premier du ou des localisateurs (20b) positionnés au niveau de l'articulation (12) ; un dispositif informatique personnel (100) comprenant un dispositif d'imagerie (614), dans lequel le dispositif informatique personnel (100) est configuré pour :

enregistrer (1402) une première image de l'articulation du patient en position d'extension complète avec le dispositif d'imagerie (614) ; enregistrer (1402) une seconde image de l'articulation du patient en position de flexion complète avec le dispositif d'imagerie (614) ; identifier (1404) des positions des localisateurs (20) de la première image et de la seconde image ; déterminer (1410) un angle d'extension de l'articulation du patient sur la base des positions identifiées des localisateurs de la première image, dans lequel l'angle d'extension est un angle entre la première ligne médiane et la seconde ligne médiane lorsque l'articulation du patient est en position d'extension complète ; déterminer (1408) un angle de flexion de l'articulation du patient sur la base des positions identifiées des localisateurs de la seconde image, dans lequel l'angle de flexion est un angle entre la première ligne médiane et la seconde ligne médiane lorsque l'articulation du patient est en position de flexion complète ; et déterminer (1412) une amplitude d'angle de mouvement sur la base de l'angle d'extension et de l'angle de flexion ; et réaliser un processus d'étalonnage pour déterminer des valeurs de décalage pour un quelconque parmi l'angle de flexion, l'angle d'extension et l'amplitude d'angle de mouvement afin de tenir compte d'une orientation du dispositif d'imagerie par rapport au membre articulé, le processus d'étalonnage comprenant :

l'analyse de la première image et de la seconde image pour déterminer une différence dans la forme des localisateurs (20) par rapport à une forme connue des localisateurs (20) ; déterminer une orientation du dispositif d'imagerie (614) par rapport au membre articulé sur la base de la différence de forme des localisateurs (20) ; et déterminer une valeur de décalage pour un quelconque parmi l'angle de flexion, l'angle d'extension et l'amplitude d'angle de mouvement afin de tenir compte de l'orientation du dispositif d'imagerie (614) par rapport au membre articulé.

**2.** Système selon la revendication 1, dans lequel le dispositif informatique personnel (100) est un dispositif mobile doté d'une application configurée pour déterminer l'amplitude d'angle de mouvement.

**3.** Système selon la revendication 1, dans lequel les positions des localisateurs (20) à la fois de la première image et de la seconde image sont identifiées sur la base des données d'image de la première image et de la seconde image.

4. Système selon la revendication 1, dans lequel le dispositif informatique personnel (100) est configuré pour générer un rapport et commander un écran d'affichage pour afficher le rapport.

5. Système selon la revendication 4, dans lequel le rapport comprend un quelconque parmi l'amplitude d'angle de mouvement, des informations historiques tabulaires de l'amplitude d'angle de mouvement, des informations historiques graphiques de l'amplitude d'angle de mouvement et des améliorations de l'amplitude d'angle de mouvement au fil du temps.

6. Système selon la revendication 4, dans lequel le dispositif informatique personnel (100) est configuré pour fournir le rapport à un dispositif clinique (612).

7. Système selon la revendication 1, dans lequel la différence de forme des localisateurs (20) est déterminée sur la base d'une ou plusieurs images enregistrées initialement.

8. Système selon la revendication 1, dans lequel le dispositif informatique personnel (100) est configuré pour fournir une notification au patient afin d'enregistrer la première image et la seconde image.

9. Système selon la revendication 1, dans lequel le système (10) comprend une source de pression négative (320) configurée pour exercer une pression négative au niveau d'une plaie (314) au niveau du membre articulé.

10. Système selon la revendication 9, dans lequel le système (10) comprend en outre un contenant (306) conçu pour recevoir l'exsudat de plaie.

11. Système selon la revendication 10, dans lequel le système (10) comprend en outre un élément de liaison (34) au niveau du champ (18) et un élément tubulaire (30), dans lequel l'élément de liaison (34) est conçu pour accoupler de manière fluide la plaie (314) avec la source de pression négative (320) par le biais de l'élément tubulaire (30, 310).

12. Système selon la revendication 1, dans lequel le système (10) comprend un pansement (36), le pansement (36) comprenant le champ (18) et une couche de collecteur (124).

FIG. 2

FIG. 1

FIG. 3

FIG. 4

FIG. 5

FIG. 6

700

702

20

21

FIG. 7

800

20

21

704

FIG. 8

900

702

20

23

FIG. 9

1000

20

704

23

FIG. 10

FIG. 11

FIG. 12

FIG. 13

FIG. 14

1500

| Establish communication between mobile patient device and a second device | 1502 |

↓

| Download installation package on mobile patient device | 1504 |

↓

| Use installation package to configure the mobile patient device to calculate range of motion angle | 1506 |

FIG. 15

1600

| Obtain initial image data from imaging device | 1602 |

↓

| Determine one or more initial parameters based on the initial image data | 1604 |

↓

| Perform process 1400 | 1606 |

↓

| Determine one or more values of the parameters based on the image data | 1608 |

↓

| Determine orientation of imaging device relative to reference point by comparing the values of the parameters to the initial parameters | 1610 |

↓

| Determine offsets/adjustments to the angles and the range of motion based on the orientaiton of the imaging device relative to the reference point | 1612 |

↓

| Offset/adjust the angles and the range of motion | 1614 |

FIG. 16

FIG. 17

FIG. 18

| $\theta_{ROM}$ | $\theta_{extend}$ | $\theta_{flexed}$ | Date: | %Imp | Total %Imp |
|---|---|---|---|---|---|
| 50 | 100 | 50 | 5/1/19 | -- | --- |
| 52 | 101 | 49 | 5/2/19 | 4% | 4% |
| 54 | 102 | 48 | 5/3/19 | 4% | 8% |
| 56 | 103 | 47 | 5/4/19 | 4% | 12% |

FIG. 19

2000

| Provide locators on dressing or skin of patient joint | 2002 |

| Perform process 1500 | 2004 |

| Perform process 1600 | 2006 |

| Generate range of motion progress report | 2008 |

| Operate display of user device to show the range of motion progress report | 2010 |

| Provide range of motion progress report to clinician device | 2012 |

## FIG. 20

2100

| Determine time since previously recorded range of motion | 2102 |

| Retrieve range of motion measurement schedule | 2104 |

| Determine next range of motion measurement time based on time since previously recorded range of motion and range of motion measurement schedule | 2106 |

| Provide reminder to patient to record range of motion data at a predetermined amount of time before the next range of motion measurement time or at the next range of motion measurement time | 2108 |

## FIG. 21

EP 4 017 355 B1

FIG. 22

35

FIG. 23

FIG. 24

FIG. 25

FIG. 26

FIG. 27

FIG. 28

Instillation Fluid Canister — 304

305

— 300

Therapy Device

— 309

— 322

Instillation Pump

P — 313

Vent — 334

— 306

— 308

— 311

Drawing Vacuum in Negative Pressure Circuit

10

Communications Interface — 324

338

Valve — 332

Removed Fluid Canister

Filter

336

Airflow

310

User Interface — 326

328

P — 330

318

320

Controller

Control Signals

Pneumatic Pump

307

Pressure Measurements

316

314

36

EP 4 017 355 B1

41

FIG. 29

EP 4 017 355 B1

FIG. 30

FIG. 31

3200

3202
Provide a dressing including a comfort layer, manifold layer, and drape at a wound

3204
Provide one or more locators on the dressing

3206
Apply negative pressure to the wound at the dressing

3208
Relieve the applied negative pressure after a time duration

3210
Perform process 2000 to calculate range of motion

3212
Re-apply negative pressure to the wound at the dressing

FIG. 32

**EP 4 017 355 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016220175 A1
- US 2017258526 A1
- US 10121066 B1

- US 7651484 B
- US 8394081 B
- US 08741813

**Non-patent literature cited in the description**

- **SU et al.** Movement of finger joints induced by synergistic wrist motion. *Clinical Biomechanics,* June 2005, vol. 20 (5